# FASCICULE DE BREVET EUROPEEN

(11) **EP 2 925 357 B1**
(45) Date de publication et mention de la délivrance du brevet: **17.07.2019**
(21) Numéro de dépôt: 13828816.2
(22) Date de dépôt: 26.11.2013
(51) Int. Cl.: A61K 39/39

(54) **MÉTHODE POUR OBTENIR DES ANTICORPS HUMAINS SPÉCIFIQUES D'UN ANTIGÈNE PAR IMMUNISATION IN VITRO**
VERFAHREN ZUR HERSTELLUNG VON ANTIGEN-SPEZIFISCHEN MENSCHLICHEN ANTIKÖRPER
METHOD TO OBTAIN ANTIGEN-SPECIFIC HUMAN ANTIBODIES USING IN VITRO IMMUNISATION

(30) Priorité: 27.11.2012 FR 1261315
(43) Date de publication de la demande: 07.10.2015
(73) Titulaire: Commissariat à l'Énergie Atomique et aux Énergies Alternatives, 75015 Paris (FR)
(72) Inventeur: LEONETTI, Michel, F-91620 Nozay (FR); LAMOURETTE, Patricia, F-91190 Gif Sur Yvette (FR); VOLLAND, Hervé, F-91400 Orsay (FR); SAVATIER, Alexandra, F-91150 Etampes (FR); WIJKHUISEN, Anne, F-91150 Etampes (FR)
(74) Mandataire: Cabinet Plasseraud
(86) Numéro de dépôt international: PCT/IB2013/060413
(87) Numéro de publication internationale: WO 2014/083499

(56) Documents cités:
- WO-A2-2011/092675

## Description

La présente invention a pour objet une méthode d'immunisation *in vitro* de lymphocytes B humains à partir d'une population totale de cellules mononuclées du sang humain périphérique, permettant d'induire la production d'anticorps humains *in vitro,* plus particulièrement d'immunoglobulines de type G (IgG) spécifiques d'un antigène (Ag). La présente invention a également pour objet une méthode de production d'anticorps humains spécifiques d'un antigène dérivée de ladite méthode d'immunisation *in vitro.*

L'obtention d'anticorps (Acs) spécifiques d'un antigène, et plus particulièrement d'IgGs, constitue un axe de recherche majeur dans le domaine thérapeutique qui découle de la démonstration de bénéfices cliniques obtenus vis-à-vis de plusieurs maladies à l'aide d'IgGs spécifiques de protéines du soi ou issues d'agent pathogènes (Reichert, J.M., mAbs, 2011, 76-99).

Afin de conserver une bonne efficacité thérapeutique et de limiter les risques de réaction secondaire inappropriée, les anticorps médicaments ne doivent pas déclencher la réaction du système immunitaire des patients. C'est pourquoi les groupes de recherche cherchent à obtenir des anticorps humains qui seront assimilés à des protéines du soi par les individus traités et seront, de ce fait, tolérés par leur système immunitaire.

Il est établi que la réponse immunitaire humorale T-dépendante dépend de la collaboration de trois partenaires cellulaires : les cellules dendritiques (CD), les lymphocytes B et les lymphocytes T CD4+ dits auxiliaires (Taux ou Th pour T-helper). La phase d'initiation de la réponse immune est localisée au niveau de la CD qui est une cellule présentatrice spécialisée (CPA, CPAg ou APC, pour *Antigen Presenting Cell*))*.* Au cours de cette phase, la CD ingère l'antigène apporté de façon exogène et certains fragments antigéniques se lient aux molécules du complexe majeur d'histocompatibilité (CMH) de classe II, les complexes peptide/molécule du CMH sont alors routés à la surface de la CD. Ces complexes sont alors reconnus par les récepteurs exprimés spécifiquement à la surface des lymphocytes Taux qui deviennent ainsi activés et contribuent à la réponse immunitaire. Le lymphocyte B reconnaît, quant à lui, l'antigène extracellulaire par l'intermédiaire de l'immunoglobuline exprimée à sa surface et l'internalise ainsi de façon plus efficace (Lanzavecchia A., Nature, 1985, 314, 537-539). Le lymphocyte B se comporte alors comme une CPAg. Il apprête l'antigène internalisé dans ses vésicules d'endocytose, les fragments d'antigène se lient alors aux molécules du complexe majeur d'histocompatibilité (CMH) de classe II puis les complexes peptides/CMH sont présentés aux lymphocytes Taux. Les lymphocytes Taux vont, en retour, aider le lymphocyte B à proliférer, à se différencier en cellule plasmocytaire sécrétrice d'anticorps et joueront un rôle crucial dans la commutation (ou *switch*) isotypique permettant l'expression d'anticorps de classe IgG. Au cours de ce processsus, plusieurs cytokines pourront contribuer au switch, notamment l'IL-21 et IL-4 (Pène et al., J. Immunol., 2004, 172, 5154-5157 ; Avery et al., J. Immunol., 2008, 181, 1767-1779). La demande internationale WO 2011/092675 décrit un complexe moléculaire de ciblage des antigènes vers les cellules présentatrices d'antigène (CPAg) qui est utilisé comme vaccin dans la prévention et le traitement des maladies infectieuses et du cancer. Le complexe moléculaire comprend une protéine Tat ou un fragment de Tat comprenant au moins la région basique Tat 49-57, associé à un antigène et à un ligand d'une molécule de surface spécifique de CPAg, la protéine Tat ou son fragment étant associé de façon covalente à l'antigène et/ou au ligand de molécule de surface de CPAg.

La réponse immunitaire humorale peut aussi être déclenchée de façon « T-indépendante ». Cette voie T-indépendante est observée avec des antigènes présentant des motifs épitopiques répétés. Les antigènes porteurs de motifs répétés vont être reconnus à la surface du lymphocyte B par plusieurs immunoglobulines qui vont se regrouper et ainsi contribuer à la mise en place d'un signal d'activation suffisant pour que la cellule sécrète des anticorps. Les anticorps produits sont principalement des IgMs car le switch isotypique est, la plupart du temps, inopérant en absence d'aide des lymphocytes Taux.

Trois approches sont généralement employées pour générer des anticorps humains (Lanzavecchia et al., Current Opinion in Biotechnology, 2007, 18, 523-528). Deux d'entre elles sont fondées sur l'isolement de lymphocytes B spécifiques sécréteurs d'anticorps obtenus à partir de sources cellulaires issues d'individus immuns pour l'Ag d'intérêt. La première approche, appelée Hu-Souris-imm, utilise comme source cellulaire des prélèvements issus de souris porteuses des loci d'immunoglobulines humaines (appelées Hu-Souris) qui sont vaccinées ou infectées par un agent pathogène. La seconde approche, appelée Hu-imm, utilise comme source cellulaire des prélèvements issus d'humains immuns pour l'Ag d'intérêt. La troisième approche, appelée Ab-display, repose sur la sélection d'anticorps à partir de banques d'anticorps exprimés sur des phages ou des levures. Ces trois voies qui ont conduit à des résultats variés présentent chacune des avantages mais aussi des limites. Ainsi, l'approche Hu-imm s'avère adaptée lorsque l'on veut obtenir des IgG spécifiques d'un agent étranger pathogène qui infecte couramment l'homme. Elle est en revanche inadéquate lorsque l'objectif est d'obtenir des anticorps contre des antigènes du soi, tels que des cytokines ou des molécules exprimées à la surface de cellules, car ces antigènes sont généralement tolérés par le système immunitaire humain afin de ne pas déclencher de réaction auto-immune. De plus, pour des agents pathogènes qui touchent rarement l'homme, comme les toxines du bioterrorisme, cette approche s'avère difficilement applicable en raison du petit nombre d'individus humains dotés d'une immunité pour ces antigènes. L'approche Hu-Souris-imm est, quant à elle, bien adaptée pour induire des réponses en IgG contre des antigènes du soi humain. Elle est en revanche inadaptée pour les agents pathogènes qui ne peuvent infecter les souris, tels que le virus de l'immunodéficience humaine, le virus de l'hépatite C, le virus de l'hépatite B ou le cytomégalovirus. De plus, la réponse immunitaire induite chez ces souris est souvent suboptimale probablement en raison de la discordance entre les IgG humaines et les récepteurs Fc de souris. L'approche Ab-display peut, quant à elle, conduire à l'isolement d'anticorps humains contre une grande variété d'antigènes. Cependant, cette approche nécessite la connaissance à priori de la cible antigénique car le principe de sélection est basé sur la liaison à un antigène purifié et pas sur un test fonctionnel. Cette contrainte de sélection conduit à exclure les tests de neutralisation virale et ne permet donc pas l'identification de nouvelles cibles neutralisantes pour des pathogènes complexes. De plus, les anticorps isolés chez la bactérie ou la levure peuvent présenter des difficultés d'expression dans les cellules de mammifère.

Les limites rencontrées dans les trois approches décrites plus haut ont conduit à rechercher des approches alternatives fondées sur l'immunisation *in vitro* de lymphocytes B humains, en particulier à partir d'une population totale de cellules mononuclées du sang humain périphérique (CMSP ou PBMC en anglais) obtenues à partir de donneurs naïfs pour la cible thérapeutique choisie, du fait que les CMSP humaines constituent une source de lymphocytes B humains renouvelable et facilement accessible.

Par rapport aux approches Hu-imm et Hu-Souris-imm décrites précédemment, cette approche présente l'avantage de ne pas nécessiter d'individus vaccinés ou infectés contre un agent pathogène. De plus, par rapport à l'approche Hu-Souris-imm, elle ne requière pas d'étape préalable d'immunisation *in vivo* et permet de se passer de l'animal.

Malgré tous les efforts déployés, la production d'anticorps humains par la technologie d'immunisation *in vitro* à partir des cellules mononuclées du sang humain périphérique (CMSP) obtenues à partir de donneurs naïfs pour la cible thérapeutique choisie, est considérée comme peu performante, peu reproductible et lourde à mettre en oeuvre.

En effet, alors que les CMSP humaines constituent une source de lymphocytes B renouvelable et facilement accessible, cette population cellulaire est reconnue comme étant la plus difficile à immuniser (Borrebaeck et al., Proc. Natl. Acad. Sci., 1988, 85, 3995-).

Il est admis que pour immuniser des CMSP humaines *in vitro,* il est absolument essentiel d'effectuer une étape préalable de déplétion des cellules inhibant la réponse immunitaire *in vitro.* Les cellules immunosuppressives sont déplétées, soit en purifiant les sous-populations de cellules B, T et dendritiques (Danielsson et al., Immunology, 1987, 61, 51-55), soit en traitant les CMSP à l'aide d'anticorps anti-CD56 et/ou anti-CD8 couplés à des billes magnétiques (Demande EP 1498426), d'agents lysosomotropes (LeuLeuOMe ; Borrebaeck et al., Biochem. Biophys. Res. Commun., 1987, 148, 941-946 ; Borrebaeck et al., Proc. Natl. Acad. Sci., 1988, 85, 3995- et Demande WO 88/01642) ou de globules rouges de mouton (méthode des rosettes ; Demande EP 0454225). L'inhibition de la production d'anticorps observée lors de l'immunisation *in vitro* de CMSP pourrait impliquer la cytokine IL-10. Cette hypothèse provient de trois observations. Premièrement, lors de l'immunisation *in vitro* , les CMSP traitées avec l'agent lysosomotrope LeuLeuOMe produisent des anticorps mais n'expriment pas le gène de l'IL-10. Deuxièmement, les CMSP non-traitées expriment le gène de l'IL-10 mais pas d'anticorps (Yamashita et al., Cytotechnology, 2007, 55, 71-77). Troisièmement, l'incubation d'anticorps anti-IL10 permet d'augmenter la production d'anticorps durant l'immunisation « in vitro ». Les traitements des CMSP utilisés pour dépléter les cellules NK présentent cependant l'inconvénient d'être toxiques et d'altérer les fonctionnalités des lymphocytes B (Mowat et al., Immunology, 1990, 69, 564-569). Les CMSP déplétées en cellules immunosuppressives sont ensuite incubées en présence de l'antigène et/ou de cytokines et/ou d'activateurs cellulaires (adjuvants, ligands de TLR, ligand de CD40, anticorps antiCD40,..). La présence de cellules B spécifiques de l'antigène capables de sécréter des anticorps est ensuite évaluée soit par dosage ELISA des surnageants de culture, soit par mesure ELISPOT des cellules sécrétrices. Ces travaux ont permis l'obtention d'IgM humaines spécifiques de différents antigènes (Borrebeack *et al.,* 1988, précité) ce qui indique que le processus de reconnaissance de l'Ag par le lymphocyte B est opérationnel et que l'action concomitante des cytokines et/ou d'autres activateurs cellulaires permet d'atteindre un niveau d'activation suffisant pour induire la sécrétion d'IgM. Cependant, le nombre de lymphocytes B sécréteurs d'IgM obtenu après fusion cellulaire ou infection avec le virus d'Epstein-Barr s'avère souvent faible (Borrebeack *et al.,* 1988, précité ; Chin et al., Immunology, 1994, 81, 428-434 ; Ishikawa et al., Cytotechnology, 1999, 31, 131-139) ce qui suggère que les méthodes d'immunisation *in vitro* qui ont été employées ne conduisent pas à l'induction de fortes réponses immunitaires. De plus, le déclenchement d'une réponse IgG est encore plus difficile à obtenir que la réponse en IgM ce qui suggère que le switch isotypique n'est pas réalisé dans les conditions expérimentales utilisées. Le faible niveau de réponse IgG a conduit au développement d'approches de culture cellulaire fondées sur des protocoles encore plus complexes dans leur réalisation. L'une de ces approches consiste à utiliser plusieurs populations cellulaires ayant subi différents processus d'activation. Ainsi, des CMSP déplétées en cellules NK et/ou CD8+ issues de personnes vaccinées contre la toxine tétanique (TT) sont incubées en présence d'un épitope T de TT (pTT) afin d'induire l'activation de lymphocytes T. Dans le même temps, des CMSP déplétées en cellules NK et/ou CD8+ et naïves pour l'Ag sont incubées en présence d'un Ag couplé à pTT (pTT-Ag) afin de déclencher une réponse primaire de type IgM. Les populations cellulaires sont ensuite mélangées en présence de fibroblastes transfectés avec CD32, d'un Ac antiCD40 et de pTT-Ag afin d'induire la réponse secondaire qui se traduit par la sécrétion d'IgG spécifiques de l'Ag (Duenas et al., Immunology, 1996, 89, 1-7). Cette approche a été encore optimisée par la suite en utilisant, d'une part, des CMSP déplétées en cellules NK, CD8+ et IL10+ pendant la phase d'induction de la réponse humorale primaire, d'autre part, le facteur de remplacement des T_{CD45RO+} lors de la phase de réponse secondaire. Ce facteur de remplacement a préalablement été obtenu à partir de CMSP déplétées en cellules NK et CD8+ puis activées avec un mitogène. Après 3 à 5 jours d'incubation, les cellules sont infectées par le virus EBV puis fusionnées avec un hétéromyélome, cette technologie complexe a permis l'obtention d'une IgG4 monoclonale spécifique du peptide antigénique (Chin et al., BMC Biotechnol, 2007, 7, 51-).

Ainsi, les méthodes d'immunisation *in vitro* utilisées jusqu'à présent s'avèrent relativement peu performantes pour sélectionner et induire des lymphocytes B humains spécifiques d'un antigène à partir de la population totale de cellules mononuclées du sang humain périphérique. De plus, les approches les plus complexes ne permettent qu'une amélioration faible du switch isotypique conduisant à l'obtention de cellules B sécrétrices d'IgGs spécifiques.

C'est pourquoi l'objectif des inventeurs a été de développer une méthode d'immunisation *in vitro* à partir des cellules mononuclées du sang humain périphérique permettant d'induire la réponse immunitaire humorale contre un antigène de façon plus simple.

Le transactivateur transcriptionnel (Tat) du virus de l'immunodéficience humaine (VIH) est une protéine qui possède des activités variées sur le système immunitaire et notamment des activités inhibitrices *in vitro.* Il a notamment été observé que Tat est capable d'induire l'apoptose des lymphocytes (Li et al., Science, 1995, 268, 429-31 ; Westendorp et al., Nature, 1995, 375, 497-500) et de perturber la réponse T spécifique qui est cruciale pour permettre le switch isotypique (Viscidi et al., Science, 1989, 246, 1606-8 ; Subramanyam et al., J. Immunol., 1993, 150, 2544-53). La protéine Tat (99 à 103 acides aminés selon les souches de VIH) comprend 5 domaines : (1) le domaine N-terminal (positions 1 à 21), qui est important pour l'interaction avec des protéines cellulaires, (2) le domaine riche en cystéines (positions 22 à 37) contenant 7 résidus de cystéine (positions 22, 25, 27, 30, 31, 34 et 37) parmi lesquelles 6 sont fortement conservées, impliqué dans la transactivation, (3) le domaine central (core) correspondant aux positions 38 à 48, également impliqué dans la transactivation, (4) le domaine basique (positions 49 à 57), qui comprend les séquences impliquées dans la localisation nucléaire, le transport transcellulaire et la liaison à l'élément de réponse TAR *(Trans-activation response)* du LTR *(Long Terminal Repeat)* viral, et qui est également impliqué dans la liaison de Tat à l'héparine, et (5) le domaine C-terminal (positions 58 à extrémité C-terminale) qui ne possède pas d'activité de transactivation mais contient le domaine riche en glutamines (positions 58 à 72) et le motif RGD (arginine-glycine-aspartate ; positions 78 à 80), nécessaire pour la liaison de Tat aux récepteurs des intégrines. En outre, Tat forme spontanément des oligomères lorsqu'elle est mise en solution alors qu'un dérivé de Tat contenant des sérines à la place des cystéines reste pleinement monomérique, ce qui indique que l'oligomérisation de Tat est médiée par les cystéines (Kittiworakarn et al., J. Biol. Chem., 2006, 281 6, 3105-3115). De plus, les mêmes auteurs ont montré que la présence d'une seule cystéine était suffisante pour former des oligomères de Tat.

Les inventeurs ont montré qu'un Ag couplé à Tat et à un ligand de CPAg est capable de déclencher une réponse immunitaire humorale *in vitro* à partir de la population totale de CMSP, c'est-à-dire sans étape préalable de déplétion des cellules cytotoxiques inhibitrices. De manière plus précise, les inventeurs ont montré que des CMSP incubées en présence de la forme Tat libre ne peuvent pas déclencher de réponse immunitaire. En revanche, elles produisent la sécrétion d'IgM et d'IgG spécifiques, quand la protéine Tat entière ou un fragment de Tat capable de s'oligomériser sont couplés à une molécule capable de lier des CPAgs. En outre, lorsque l'on utilise Tat couplée à une molécule capable de lier les CPAgs pour induire la réponse humorale, l'intensité de la réponse en Acs spécifiques obtenue avec la population totale de CMSP est comparable à celle obtenue avec la population de CMSP déplétées en cellules NK. Enfin, les inventeurs ont également montré qu'un Ag différent de Tat ou d'un fragment antigénique de Tat peut aussi déclencher la réponse immunitaire en Acs spécifiques *in vitro,* en particulier d'IgGs spécifiques, à partir de la population totale de CMSP, quand il est préalablement couplé à Tat et à une molécule capable de lier les CPAgs.

L'invention est telle que définie dans les revendications.

En conséquence, la présente invention a pour objet une méthode d'immunisation *in vitro* de lymphocytes B humains, comprenant la culture d'une population totale de cellules mononuclées du sang humain périphérique (CMSP ou CMSP humaines) en présence d'une composition antigénique comprenant au moins un antigène lié de façon covalente à la fois à :
(i) une protéine Tat ou à un fragment de Tat capables de s'oligomériser par l'intermédiaire de cystéine(s) de la région riche en cystéines de Tat et
(ii) un ligand d'une molécule de surface spécifique des cellules présentatrices d'antigène,
le fragment de Tat capable de s'oligomériser comprenant les régions riche en cystéines, core et basique, et
le ligand de ladite molécule de surface spécifique des cellules présentatrices d'antigène étant sélectionné dans le groupe comprenant : la protéine BB, la protéine ZZ, un anticorps anti-CMH de classe II, anti-RFcgamma, anti-IgM, anti-IgD, anti-IgG, anti-DEC-205, anti-CD209 ou anti-CD20 et un fragment des anticorps précédents comprenant au moins le paratope.

La méthode d'immunisation *in vitro* de lymphocytes B humains de l'invention présente les avantages suivants par rapport aux méthodes de l'art antérieur :
- elle permet de déclencher efficacement des réponses en IgGs spécifiques de l'Ag, et
- l'immunisation *in vitro* est plus simple étant donné qu'elle est réalisée avec une population totale de cellules mononuclées du sang humain périphérique alors que les autres méthodes nécessitent l'élimination des cellules immunosuppressives par des traitements supplémentaires qui diminuent l'efficacité d'obtention de lymphocytes B immunisés producteurs d'anticorps spécifiques de l'Ag, du fait qu'ils sont toxiques et modifient la fonctionnalité des lymphocytes B.

### Définitions

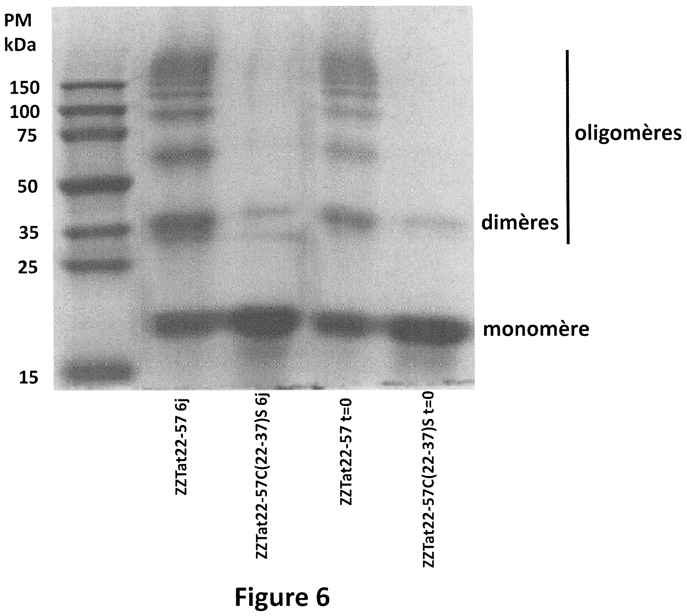

- - On entend par « immunisation *in vitro* de lymphocytes B humains », l'induction d'une réponse humorale *in vitro,* c'est-à-dire la production *in vitro* d'anticorps humains spécifiques d'un antigène qui résulte de la reconnaissance dudit antigène par les immunoglobulines exprimées à la surface de lymphocytes B humains naïfs mis en culture, *in* vitro avec l'antigène.
- On entend par « lymphocytes B naïfs », les lymphocytes B qui n'ont jamais rencontré l'antigène qu'ils pourraient lier via le paratope exprimé par leur immunoglobuline de surface. Ces lymphocytes B sont issus directement du sang périphérique d'un donneur n'ayant jamais été en contact avec l'antigène. Ces donneurs présenteront donc un statut séronégatif pour ledit antigène, c'est-à-dire qu'ils présenteront un taux indétectable d'anticorps sériques spécifiques dudit antigène.
- On entend par « antigène » toute substance pouvant être reconnue spécifiquement par le système immunitaire et en particulier par les anticorps et les cellules du système immunitaire adaptatif (lymphocytes B, lymphocytes T CD4+, lymphocytes T CD8+).
- On entend par « population totale de cellules mononuclées du sang humain périphérique, population totale de CMSP, CMSP, PBMC, CMSP ou PBMC totales, CMSP ou PBMC humaines », l'ensemble des cellules mononuclées présentes dans le sang humain périphérique, c'est-à-dire la population constituée principalement de lymphocytes (B, T, NK) et de monocytes, qui peut être isolée du sang humain total par les techniques classiques telles que la centrifugation en gradient de densité.
- On entend par « protéine Tat » ou « Tat », une protéine comprenant la séquence en acides aminés de la protéine Tat d'un isolat du virus de l'immunodéficience humaine (VIH ; séquence naturelle) ou une séquence synthétique dérivée d'une ou plusieurs séquences naturelles de Tat et correspondant à une protéine Tat possédant des caractéristiques structurales et fonctionnelles semblables à celles d'une protéine Tat du VIH.
- On entend par « protéine Tat ou fragment de Tat capables de s'oligomériser », une protéine Tat ou un fragment peptidique de Tat (peptide de Tat), capable de s'autoassocier, c'est-à-dire de s'assembler avec une ou plusieurs protéines ou fragments identiques pour former une molécule oligomérique constituée d'au moins deux desdites protéines ou fragments comme illustré dans la figure 6 pour le peptide Tat 22-57.
- On entend par « cellule présentatrice d'antigène(s) », cellule présentatrice de l'antigène, CPA, CPAg ou APC pour *Antigen Presenting Cell,* une cellule exprimant une ou plusieurs molécules du complexe majeur d'histocompatibilité (CMH) de classe I et de classe II (molécules HLA de classe I et de classe II chez l'homme) et capable de présenter les antigènes aux lymphocytes T CD4+ et T CD8+ spécifiques de cet antigène. Comme cellules présentatrices d'antigène, on peut citer notamment les cellules dendritiques (CD ou DC pour *Dendritic Cell*), les cellules mononuclées du sang périphérique (CMSP ou PBMC en anglais), les monocytes, les macrophages, les lymphocytes B, les lignées lymphoblastoïdes, et les lignées de cellules humaines ou animales génétiquement modifiées exprimant des molécules du CMH de classe I et de classe II, notamment des molécules HLA I et HLA II.
- On entend par « molécule de surface de cellules présentatrices d'antigène », une molécule exprimée à la surface de cellules présentatrices d'antigène.
- On entend par « molécule de surface spécifique des cellules présentatrice d'antigène », une molécule de surface exprimée uniquement sur les CPAgs ou une molécule exprimée essentiellement sur les cellules présentatrices d'antigène, c'est-à-dire une molécule de surface exprimée sur les CPAgs ainsi que sur un nombre très limité de cellules autres que les CPAgs, et possédant de ce fait une spécificité d'expression élevée pour les CPAgs, *i.e.* une molécule quasi-spécifique des CPAgs.
- On entend par « récepteur d'endocytose », un récepteur capable de médier l'endocytose d'un ligand de ce récepteur.
- On entend par anticorps (Ac), une immunoglobuline de classe ou d'isotype IgG, IgM, IgA, IgD ou IgE.
- On entend par anticorps spécifique, un anticorps dirigé contre une molécule particulière, communément appelée antigène.
- On entend par épitope B, la région d'un antigène qui est reconnue par un anticorps (épitope B) spécifique de cet antigène. L'épitope peut notamment être constitué par un peptide (peptide épitope). Il peut aussi être constitué par le repliement de plusieurs zones éloignées dans la séquence d'une protéine. Il peut aussi être constitué par de petites structures, appelées haptènes, qui sont incapables de déclencher la réponse immunitaire à elles seules.
- On entend par épitope T, la région d'un antigène qui est reconnue par un lymphocyte T CD4+ (épitope T CD4+) ou un lymphocyte T CD8+ (épitope T CD8+) spécifique de cet antigène. L'épitope peut notamment être constitué par un peptide (peptide épitope).

Conformément à l'invention, l'immunisation *in vitro* des lymphocytes B est réalisée à partir de lymphocytes B naïfs, c'est-à-dire issus d'individu(s) séronégatif(s) pour ledit antigène. L'absence d'anticorps sériques spécifiques de l'antigène chez le(s) donneur(s) est vérifiée par un test classique, notamment un test ELISA.

La population de CMSP est obtenue à partir d'un prélèvement de sang humain périphérique, selon les techniques classiques d'isolement des cellules du système immunitaire, en suivant les protocoles standards connus de l'Homme du métier.

La population totale de CMSP est généralement isolée par la technique de centrifugation en gradient de densité, en particulier sur gradient de Ficoll®.

Les cellules mononuclées isolées du sang humain périphérique sont ensuite mises en culture dans les conditions standards (milieu, température, CO₂) de culture des cellules du système immunitaire, connues de l'homme du métier.

Les CMSP sont cultivées en présence d'une composition antigénique comprenant au moins l'antigène (Ag) lié de façon covalente à la fois à : (i) une protéine Tat ou à un fragment de Tat capables de s'oligomériser (Tat), et (ii) un ligand d'une molécule de surface spécifique des cellules présentatrices d'antigène (Ligand, ligand, Ligand de CPAg, ou ligand de CPAg).

La composition antigénique comprend généralement trois éléments distincts, l'Ag, Tat ou le fragment de Tat capables de s'oligomériser et le Ligand de CPAg, étant donné que l'Ag est généralement différent de Tat ou d'un fragment antigénique de Tat inclus dans ladite protéine Tat ou ledit fragment de Tat capables de s'oligomériser. Toutefois, lorsque l'Ag est Tat ou un fragment antigénique de Tat inclus dans ladite protéine Tat ou ledit fragment de Tat capables de s'oligomériser, alors la composition antigénique ne comprend que deux éléments distincts, Tat ou le fragment de Tat capables de s'oligomériser et le Ligand de CPAg.

La liaison covalente est notamment générée par un couplage chimique covalent (formation d'un conjugué covalent) ou par la construction d'une protéine de fusion (fusion génétique). L'Ag, Tat et le Ligand sont liés, soit uniquement par des couplages chimiques covalents ou des fusions génétiques, soit par un mélange des deux. En outre, la liaison de l'Ag avec Tat et le Ligand peut être directe ou indirecte, c'est-à-dire que l'Ag est lié directement à Tat et au Ligand (Tat-Ag-Ligand) ou Tat et le Ligand sont liés de façon covalente (Tat-Ligand) et l'Ag est lié de façon covalente, soit à Tat (Ag-Tat-Ligand), soit au ligand (Tat-Ligand-Ag).

L'antigène est un antigène naturel, recombinant ou synthétique, qui peut correspondre à une molécule du soi ou à un agent pathogène atténué ou inactivé (virus, bactérie, parasite, champignon). L'antigène peut correspondre à une particule virale synthétique, une molécule isolée (protéine, polysaccharide, lipide, lipoprotéine, glycoprotéine ou lipopolysaccharide) ou un fragment de molécule comprenant un ou plusieurs épitopes B, éventuellement associés à un ou plusieurs épitopes T CD4+ , notamment sous forme d'un peptide ou d'un polypeptide comprenant des épitopes issus d'un seul antigène ou de plusieurs antigènes différents (fragment polyépitopique).

De préférence l'antigène est une cible pour le diagnostic ou le traitement d'une maladie, de préférence choisie parmi les cancers, les maladies auto-immunes, les maladies causées par des agents pathogènes (virus, bactéries, parasites, champignons, ..), agents de maladies infectieuses ou de bioterrorisme) ou par leurs toxines (toxine botulique, ricine, anthrax, ..), les maladies inflammatoires chroniques et le rejet de greffe.

La molécule de surface spécifique des CPAgs qui est ciblée par le ligand est une molécule de surface exprimée essentiellement sur les CPAgs et en particulier sur les lymphocytes B. De préférence, ladite molécule de surface des CPAgs est un récepteur d'endocytose. Parmi ces molécules de surface, on peut citer notamment : les molécules du CMH classe II ; les immunoglobulines de surface ou immunoglobulines membranaires ; les IgG en interaction avec le ou les récepteurs pour la région constante des immunoglobulines, FcγR: FcγRI (CD64), FcyRII (CD32), FcyRIII (CD16)) ; CD20 ; les récepteurs des lectines de type C, DEC-205(CD205) et DC-SIGN (CD209) et les récepteurs pour la région constante des immunoglobulines, FcyR : FcyRI (CD64), FcyRII (CD32), FcyRIII (CD16)).

Selon un autre mode de mise en oeuvre avantageux de l'invention, la molécule de surface spécifique des CPAgs qui est ciblée par le ligand est sélectionnée dans le groupe constitué par: les immunoglobulines membranaires, notamment les IgD et les IgM membranaires ; les récepteurs pour la région constante des immunoglobulines, FcyR ; les IgG en interaction avec le ou les FcyR ; les molécules du CMH de classe II ; CD20, et les récepteurs des lectines de type C, DEC-205(CD205) et DC-SIGN (CD209).

Le ligand est notamment le fragment BB (protéine BB ou double domaine BB) de la protéine A de *S. aureus* et son dérivé ZZ (protéine ZZ ou double domaine ZZ), le fragment BB liant les régions Fc et le Fab des immunoglobulines alors que le double domaine ZZ ne lie que la région Fc. La protéine BB correspond à la séquence SEQ ID NO : 1 et la protéine ZZ à la séquence SEQ ID NO : 2. Le ligand est également une protéine d'enveloppe d'un virus (VIH, virus de la dengue, virus sindbis,...) qui utilise ces molécules de surface des CPAgs comme récepteur d'endocytose. Alternativement, le ligand est un anticorps naturel ou recombinant dirigé contre ces molécules de surface des CPAgs ou un fragment de cet anticorps contenant au moins le paratope (domaine de liaison à l'antigène), tels qu'un fragment Fab, Fab', F(ab')₂, Fv ou Fv simple chaîne (scFv pour *single-chain Fv*), Fabc, ou un fragment Fab comprenant une portion du domaine Fc.

De plus l'antigène est éventuellement associé, de façon covalente ou non covalente, à d'autres ligands de molécules de surface de CPAgs, notamment des ligands qui ciblent d'autres molécules de surface de CPAgs que le premier ligand (L1), en particulier des molécules de surface présentes sur d'autres CPAgs que celle ciblée par le premier ligand. La liaison non-covalente des autres ligands (L2, L3,..) est obtenue par tout moyen connu de l'Homme du métier. Elle peut notamment être obtenue en utilisant une molécule (élément de liaison), notamment un peptide, possédant une affinité élevée et spécifique pour L1, Tat, l'Ag ou L2. Cet élément est lié de façon covalente à L1, L2, Tat ou l'Ag. L'affinité de l'élément de liaison pour son partenaire, dans le complexe est suffisante pour qu'il ne se dissocie pas immédiatement de ce complexe *in vitro.* Lorsque L1 ou L2 sont des anticorps, l'élément de liaison est notamment une protéine ou un fragment de protéine qui lie la région Fc et/ou Fab des immunoglobulines, tels que définis ci-dessus. En outre, lorsque L1 ou L2 est une protéine ou un fragment de protéine qui lie la région Fc et/ou Fab des immunoglobulines, tels que définis ci-dessus, il joue le rôle d'élément de liaison pour l'autre ligand qui est un anticorps.

Le ligand de ladite molécule de surface spécifique des CPAgs est sélectionné dans le groupe constitué par ou comprenant : la protéine BB, la protéine ZZ , un anticorps anti-CMH de classe II, un anticorps anti-IgD, anti-IgG ou anti-IgM, un anticorps anti-RFcgamma (I, II et/ou III), notamment anti-CD32, un anticorps anti-DEC-205, anti-CD209 ou anti-CD20, et un fragment des anticorps précédents comprenant au moins le paratope, notamment un fragment Fab, Fab', F(ab')₂, Fv, scFv, Fabc ou Fab comprenant une portion de la région Fc.

L'invention englobe l'utilisation d'une protéine Tat naturelle ou synthétique, notamment un variant de Tat obtenu par l'insertion, la substitution et/ou la délétion d'un ou plusieurs acides aminés de Tat, ou d'un fragment de ladite protéine ou dudit variant capables de s'oligomériser et d'induire une immunisation *in vitro* spécifiques de Tat lorsqu'ils sont couplés de façon covalente à un Ligand de CPAg tel que défini ci-dessus, notamment à la protéine ZZ.

Un exemple de protéine Tat est la séquence SEQ ID NO: 3 qui est celle de l'isolat NDK du VIH-1 qui correspond à une séquence consensus précédemment obtenue à partir de séquences d'isolats primaires de VIH-1 rapportées dans les bases de données.

L'oligomérisation de la protéine Tat ou du fragment de Tat peut être réalisée avec une protéine Tat ou un fragment de Tat modifiés ou non-modifiés. L'oligomérisation est réalisée en utilisant des cystéines mais elle peut également être réalisée en utilisant tout type de résidu d'acide aminé et de modification permettant le couplage covalent entre deux peptides ou protéines, qui sont bien connus de l'Homme du métier. A titre d'exemple non-limitatif, on peut citer les cystéines modifiées, notamment les résidus pénicillamines, ainsi que les résidus respectivement fonctionnalisés avec un azoture et un alcyne qui se couplent par chimie click, et les résidus fonctionnalisés avec des alcènes qui se couplent par réaction de métathèse.

Ladite protéine Tat ou ledit fragment de Tat s'oligomérise par l'intermédiaire de cystéine(s). Il s'agit de cystéine(s) de la région riche en cystéine(s) (C22, C25, C27, C30, C31, C34 et/ou C37, en référence à la séquence SEQ ID NO : 3).

Ledit fragment de Tat comprend les régions riche en cystéines (22-37), core (38-48) et basique (49-57).

Des fragments de Tat conformes à l'invention incluent le peptide Tat 22-57 (lesdites positions étant indiquées en référence à la séquence SEQ ID NO : 3) et les variants dudit peptide possédant une à six des cystéines C22, C25, C27, C30, C31, C34 et C37, les cystéines restantes étant substituées par un autre acide aminé, notamment une sérine ou une alanine.

Selon une disposition avantageuse des modes de mise en oeuvre précédents, la composition antigénique comprend au moins un Ag lié de façon covalente à (i) une protéine Tat ou un fragment de Tat capables de s'oligomériser et (ii) la protéine BB ou la protéine ZZ qui lient la région Fc et/ou Fab des immunoglobulines. Le fragment de Tat est de préférence choisi parmi Tat 22-57 et les variants dudit peptide possédant une à six des cystéines C22, C25, C27, C30, C31, C34 et C37, les cystéines restantes étant substituées par un autre acide aminé, notamment une sérine ou une alanine. De préférence, il s'agit d'une protéine de fusion entre l'Ag, Tat ou le fragment de Tat et la protéine ZZ ou la protéine BB. De préférence, ladite composition comprend également un anticorps anti-CMH de classe II, un anticorps anti-RFcgamma (I, II et/ou III), notamment un anticorps anti-CD32, un anticorps anti-IgD, anti-IgG ou anti-IgM, un anticorps anti-DEC-205, anti-CD209 ou anti-CD20, ou un fragment des anticorps précédents comprenant au moins le paratope, notamment un fragment Fab, Fab', F(ab')₂, Fv, scFv, Fabc ou Fab comprenant une portion de la région Fc, lié de façon non-covalente à ladite protéine ou ledit fragment de protéine qui lie la région Fc et/ou ou Fab des immunoglobulines.

Selon une autre disposition avantageuse des modes de mise en oeuvre précédents, la composition antigénique comprend au moins un Ag lié de façon covalente à (i) une protéine Tat ou un fragment de Tat capables de s'oligomériser et (ii) un anticorps anti-CMH de classe II, un anticorps anti-RFcgamma (I, II et/ou III), notamment un anticorps anti-CD32, un anticorps anti-IgD, anti-IgG ou anti-IgM, un anticorps anti-DEC-205, anti-CD209 ou anti-CD20, ou un fragment des anticorps précédents comprenant au moins le paratope, notamment un fragment Fab, Fab', F(ab')₂, Fv, scFv, Fabc ou Fab comprenant une portion de la région Fc. Le fragment de Tat est de préférence choisi parmi Tat 22-57 et les variants dudit peptide possédant une à six des cystéines C22, C25, C27, C30, C31, C34 et C37, les cystéines restantes étant substituées par un autre acide aminé, notamment une sérine ou une alanine.

Selon encore une autre disposition avantageuse des modes de mise en oeuvre précédents, la composition antigénique comprend au moins un Ag lié de façon covalente à (i) une protéine Tat ou un fragment de Tat capables de s'oligomériser et (ii) au fragment BB de la protéine A de *Staphylococcus aureus* qui lie la région Fc et/ou Fab des anticorps et (iii) un anticorps (non-spécifique ou spécifique dudit antigène), de préférence une IgG, ou un fragment dudit anticorps comprenant au moins la région Fc. Le fragment de Tat est de préférence choisi parmi Tat 22-57 et les variants dudit peptide possédant une à six des cystéines C22, C25, C27, C30, C31, C34 et C37, les cystéines restantes étant substituées par un autre acide aminé, notamment une sérine ou un alanine.

Selon un autre mode de mise en oeuvre avantageux de l'invention, la composition antigénique comprend au moins un facteur d'activation des lymphocytes B, et/ou un facteur de différenciation des lymphocytes B, notamment un facteur capable d'induire la commutation isotypique et la production d'autres isotypes d'immunoglobulines (IgA, IgG). Les facteurs capables d'induire la commutation isotypique sont notamment choisis parmi une ou plusieurs des cytokines IL-1, IL-2, IL-4, IL-5, IL-6, IL-13, IL-21 et TGF-béta. De préférence ladite composition comprend l'IL-21 et éventuellement l'IL-4 et/ou un ligand de CD40 tel qu'un anticorps anti-CD40 ou le ligand soluble de CD40, appelé CD40L.

La composition antigénique est sous forme soluble ou particulaire. Lorsque la composition est sous forme particulaire, l'Ag lié à Tat et au(x) Ligand(s), et éventuellement les autres composants, sont incorporés à l'intérieur ou à la surface d'une particule du type liposome, virosome, nanoparticule, microsphère solide (polymère, silice) ou mixte.

La méthode d'immunisation *in vitro* selon la présente invention permet d'obtenir des lymphocytes B humains immunisés avec l'antigène. Ces lymphocytes B humains immunisés produisent des anticorps humains spécifiques d'isotype IgG, IgM, IgA, et/ou IgE, qui sont sécrétés dans le milieu de culture.

Toutefois, dans la mesure où les lymphocytes B ont une durée de vie limitée, il est nécessaire de les immortaliser afin d'obtenir une production continue d'anticorps spécifiques de l'antigène.

En conséquence, la présente invention a pour objet une méthode de production d'anticorps humains spécifiques d'un antigène, comprenant :
a) l'immunisation *in vitro* de lymphocytes B humains avec un antigène (Ag) lié de façon covalente à la fois à : (i) une protéine Tat ou à un fragment de Tat capable de s'oligomériser (Tat), et (ii) un ligand d'une molécule de surface spécifique des cellules présentatrices d'antigène (Ligand ou Ligand de CPAg), selon la méthode d'immunisation *in vitro* de lymphocytes B humains de l'invention telle que décrite ci-dessus,
b) l'immortalisation des lymphocytes B immunisés obtenus à l'étape a), et
c) la récupération des anticorps humains spécifiques de l'antigène produits par les lymphocytes B immortalisés obtenus à l'étape b).

Les anticorps produits selon la méthode de production in vitro d'anticorps de l'invention sont des anticorps monoclonaux ou polyclonaux d'isotype IgG, IgM, IgA, IgE, spécifiques d'un antigène tel que défini ci-dessus.

L'immortalisation des lymphocytes B est réalisée selon les techniques classiques connues de l'Homme du métier. Elle peut être effectuée par infection avec un virus transformant tel que le virus Epstein-Barr (EBV ; Lanzavecchia et al., Current Opinion in Biotechnology, 2007, 18, 523-538), de préférence l'infection par EBV est réalisée en présence d'un agoniste du TLR tel qu'un motif CpG (Traggiai et al., Nature Medicine, 2004, 10, 871-875). Alternativement, l'immortalisation est réalisée par fusion des lymphocytes B avec un myélome, notamment un myélome humain ou murin, une lignée de cellules lymphoblastoïdes, des cellules de lymphome ou une lignée d'hétéromyélome selon les techniques classiques de fusion cellulaire. De nombreux partenaires de fusion des lymphocytes B permettant l'obtention d'hybridomes producteurs d'anticorps monoclonaux spécifiques de l'antigène ont été décrits. On peut citer notamment les lignées CB-F7, B6B11, CB-F7, K6H6/B5, H7NS d'hétéromyélome humain/souris (vor dem Esche et al., Immunobiology, 2011, 216, 847-853 ; Kalantarov et al., Human Antibodies, 2002, 11, 85-96 ; Delvig et al., Human Antibodies Hybridomas, 1995, 6, 42-46) ; la lignée MFP-2 de triome résultant de la fusion de l'hétéromyélome B6B11 avec un lymphocyte de ganglion lymphatique (Kalantarov et al., Human Antibodies, 2002, 11, 85-96). En outre, on peut immortaliser les lymphocytes B en combinant l'infection avec EBV et la fusion avec un partenaire cellulaire tel que défini ci-dessus.

Après l'étape d'immortalisation, les lymphocytes producteurs d'anticorps spécifiques de l'antigène sont identifiés par les techniques classiques connues de l'Homme du métier, notamment par ELISA, puis ils sont généralement clonés par les méthodes standards telles que la technique de la dilution limite, de façon à obtenir des lymphocytes B producteurs d'un anticorps monoclonal spécifique de l'antigène. Les anticorps spécifiques de l'antigène produits par les lymphocytes B qui sont sécrétés dans le milieu de culture sont récupérés par simple prélèvement du surnageant de culture. Ensuite, les anticorps sont généralement purifiés selon les techniques classiques connues de l'Homme du métier, telles que par exemple la chromatographie d'affinité.

Selon un mode de mise en oeuvre avantageux de l'invention, ladite méthode de production d'anticorps comprend une étape supplémentaire de clonage des lymphocytes B immortalisés, entre les étapes b) et c). Selon un autre mode de mise en oeuvre avantageux de l'invention, lesdits anticorps spécifiques de l'antigène sont des anticorps humains d'isotype IgG.

Selon un autre mode de mise en oeuvre avantageux de l'invention, il s'agit d'anticorps monoclonaux humains, de préférence d'isotype IgG.

La description divulgue un kit pour la mise en oeuvre de la méthode d'immunisation *in vitro* ou de la méthode de production d'anticorps selon l'invention. Le kit comprend une composition antigénique telle que définie ci-dessus, incluant notamment le peptide Tat 22-57 ou à un variant dudit peptide possédant une à six des cystéines C22, C25, C27, C30, C31, C34 et C37, les cystéines restantes étant substituées par un autre acide aminé, notamment une sérine ou une alanine.

La composition antigénique selon l'invention est préparée par les techniques classiques connues de l'Homme du métier, à savoir :
- l'antigène, et les ligands des molécules de surface des CPAgs peuvent être produits par synthèse chimique ou par expression d'un ADN recombinant dans un système cellulaire approprié, eucaryote ou procaryote. Les peptides et protéines peuvent être synthétisés en phase solide, selon la technique Fmoc, originellement décrite par Merrifield et al. (J. Am. Chem. Soc., 1964, 85, 2149-) et purifiés par chromatographie liquide haute performance en phase inverse. Les polypeptides et les protéines peuvent être produits à partir des ADNc correspondants, clonés dans un vecteur d'expression eucaryote ou procaryote approprié, les polypeptides ou protéines produits dans les cellules modifiées par le vecteur recombinant sont purifiées par tout moyen approprié, notamment par chromatographie d'affinité. Des anticorps dirigés contre des molécules de surface de CPAgs sont bien connus et disponibles commercialement. Par exemple, à titre d'exemple non-limitatif, les anticorps anti-CD205 (#555831) ; anti-CD206 (#555952) ; anti-CD209 (#551186) ; anti-HLA-DR (#555556) sont disponibles chez BECTON-DICKINSON. Alternativement, des anticorps monoclonaux peuvent être produits par les techniques classiques connues de l'homme du métier. Par exemple, les anticorps monoclonaux sont produits à partir d'hybridomes obtenus par fusion de lymphocytes B d'un animal immunisé par la molécule de surface de CPAgs avec des myélomes, selon la technique de Köhler et Milstein (Nature, 1975, 256, 495-497) ; les hybridomes sont cultivés *in vitro,* notamment dans des fermenteurs ou produits *in vivo,* sous forme d'ascite; alternativement lesdits anticorps monoclonaux sont produits par génie génétique comme décrit dans le brevet américain US 4,816,567. Les anticorps humanisés sont produits par des méthodes générales comme celles décrites dans la Demande Internationale WO 98/45332. Les fragments d'anticorps sont produits à partir des régions V_{H} et V_{L} clonées, à partir des ARNm d'hybridomes ou de lymphocytes d'un mammifère immunisé; par exemple, les fragments Fv, scFv ou Fab sont exprimés à la surface de phages filamenteux selon la technique de Winter et Milstein (Nature, 1991, 349, 293-299); après plusieurs étapes de sélection, les fragments d'anticorps spécifiques de l'antigène sont isolés et exprimés dans un système d'expression approprié, par les techniques classiques de clonage et d'expression d'ADN recombinant. Les anticorps ou leur fragments tels que définis ci-dessus, sont purifiés par les techniques classiques connues de l'Homme du métier, telles que la chromatographie d'affinité.
- l'association covalente de l'antigène (Ag) à Tat ou au fragment de Tat (Tat) et aux ligand (s) de molécules de surface de CPAgs (Ligand(s)) peutêtre réalisé par la construction d'une protéine de fusion dans laquelle les séquences nucléotidiques codant pour l'Ag, Tat et le(s) ligand(s) sont fusionnées en phase, dans l'ordre approprié, soit directement, soit par l'intermédiaire d'une séquence nucléotidique codant pour un peptide de liaison (*peptide linker*) approprié. En fonction des tailles respectives des séquences en acides aminés de l'Ag, Tat et le(s) ligand(s), elles sont soit fusionnées au niveau de leurs extrémités (extrémité N-terminale de l'une des séquences fusionnée à l'extrémité C-terminale de l'autre séquence) ou l'une des séquences est insérée dans l'autre séquence à un site approprié qui n'a pas d'effet délétère sur l'immunogénicité de l'antigène et la liaison du ou des ligands à son récepteur exprimé à la surface des CPAgs. Alternativement, l'Ag, Tat et le(s) ligand(s) peuvent être couplés de façon covalente, par tout moyen approprié. Le couplage est effectué par l'intermédiaire de groupements réactionnels initialement présents ou préalablement introduits sur l'antigène, Tat et le(s) ligand(s). Le couplage peut notamment être réalisé au niveau de résidus d'acides aminés dont la chaîne latérale comprend une fonction réactive. Parmi ces acides aminés, on peut citer les acides aminés polaires comprenant une fonction : -OH [sérine (S), thréonine (T) ou tyrosine (Y)], -SH [cystéine (C)], -NH₂ [lysine (K) ou arginine (R)], -COOH [acide aspartique (D) ou acide glutamique (E)], et les acides aminés polaires à chaîne latérale fonctionnalisée par addition d'une fonction réactive. L'antigène est couplé à Tat et/ou aux ligand(s) par tout moyen approprié ; ces moyens qui sont connus de l'Homme du métier incluent notamment le couplage à l'aide de réactifs homobifonctionnels comme le glutaraldéhyde ou le dithiobis-(succinimidyl propionate). De préférence, le couplage est réalisé à l'aide de réactifs hétérobifonctionnels, notamment le m-maleimido-benzoyl-N-hydroxysuccinimide (SMCC) ou le sulfo-SMCC qui contiennent chacun un groupement maléimide capable de réagir avec les thiols libres. Dans ce cas, le SMCC est préalablement lié de façon covalente à une fonction amine présente sur l'Ag, Tat ou le(s) ligand(s). Dans le même temps, un autre réactif hétérobifonctionnel (comme le N-succinimidyl S-acetylthioacétate qui contient un groupement thio-ester clivable à l'hydroxylamine ou le succinimidyl-pyridyl-dithiopropionate, qui contient un pont disulfure réductible en conditions douce), est associé à une fonction amine du second partenaire qui est l'Ag , Tat ou un des ligands. Le second partenaire est ensuite traité à l'hydroxylamine ou avec un réducteur pour permettre la libération du thiol. Le composé thiolé est alors incubé avec le composé ayant incorporé le maléimide et le couplage est obtenu par réaction du groupement thiol sur le groupement maléimide. Ce type de couplage covalent est notamment décrit dans Léonetti et al., J. Exp. Med., 1999, 189, 1217-1228. Il est aussi possible de libérer un groupement thiol déjà présent sur un des composés pour ensuite réaliser son couplage à un autre composé qui a été préalablement modifié à l'aide de SMCC. Cette méthode, qui est souvent employée pour coupler des anticorps à des ligands, est notamment décrite dans Ishikawa et al., J. Immunoassay, 1983, 4, 209-327.
- les complexes non-covalents sont préparés par mise en contact du deuxième ligand (L2) avec l'antigène lié de façon covalente au premier ligand et à Tat (L1-Ag-Tat, l'ordre de L1 et de l'Ag étant indifférent lorsqu'il s'agit d'une protéine de fusion), dans des conditions permettant aux deux partenaires d'interagir. Cette interaction peut impliquer un élément de liaison, notamment une protéine ou un peptide, qui possède une affinité élevée et spécifique, pour l'un des partenaires du complexe (Ag, Tat ou L1). En particulier, l'affinité de l'élément de liaison pour ce partenaire, dans le complexe, est suffisante pour qu'il ne se dissocie pas immédiatement de ce complexe *in vitro.* Lorsque l'un des ligands est une immunoglobuline, l'élément de liaison est un élément de liaison aux immunoglobulines tel que décrit dans la Demande FR 2759296. Lorsque l'un des ligands est une immunoglobuline ou un fragment d'immunoglobuline et l'autre une protéine ou un fragment de protéine qui lie les immunoglobulines, L1 et L2 peuvent s'associer directement.

La mise en oeuvre de l'invention utilise, sauf indication contraire, des méthodes classiques d'immunologie, de culture cellulaire, de biologie cellulaire, de biologie moléculaire et d'ADN recombinant qui sont connues de l'homme du métier.

Outre les dispositions qui précèdent, l'invention comprend encore d'autres dispositions qui ressortiront de la description qui va suivre, qui se réfère à des exemples de mise en oeuvre de la méthode objet de la présente invention ainsi qu'aux dessins annexés, dans lesquels :

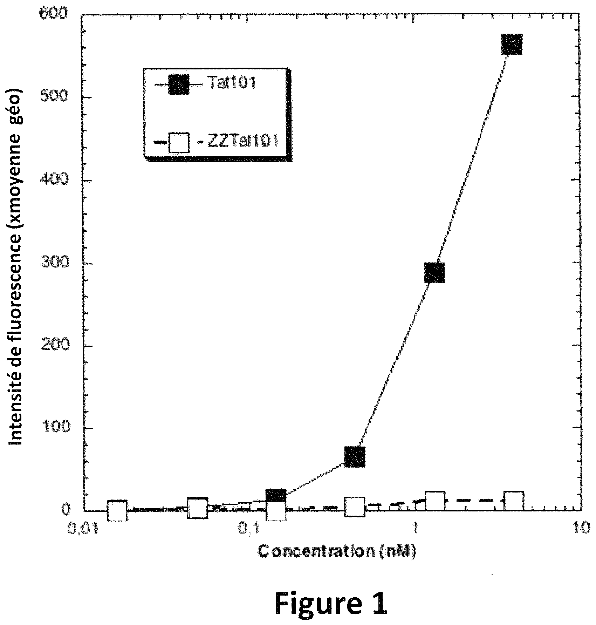

- la figure 1 montre que l'activité de transactivation de la protéine Tat101 est altérée quand elle est fusionnée au double domaine ZZ. Une lignée HeLa transfectée de façon stable avec un plasmide codant pour la séquence LTR du VIH-1 et pour la séquence de la protéine EGFP a été incubée avec la protéine Tat101 libre ou fusionnée à ZZ (ZZ-Tat101) pendant 45 heures à 37°C. L'expression de l'EGFP a ensuite été examinée par cytométrie de flux (FACS).

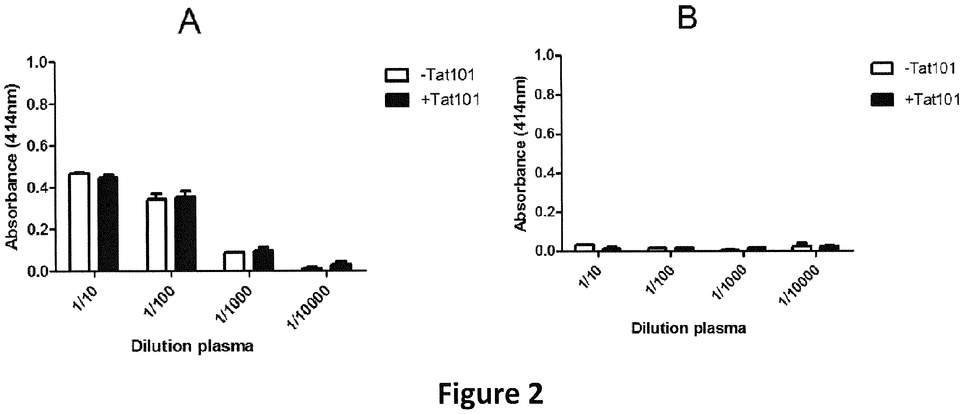

- la figure 2 illustre la mesure des anticorps spécifiques anti-Tat101 avant l'immunisation *in vitro* de CMSP humaines. A. La réponse en IgM anti-Tat101 dans les plasmas individuels des donneurs de sang (EFS Rungis, France). La réponse IgM anti-Tat101 a été évaluée par un test immuno-enzymatique. Des séries de dilutions de plasma ont été incubées toute la nuit dans des plaques adsorbées avec Tat101, en présence ou en absence de Tat101 comme compétiteur (2 µg/ml de concentration finale). **B**. La réponse en IgG anti-Tat101 dans les plasmas individuels des donneurs de sang (EFS Rungis, France). La réponse IgG anti-Tat101 a été évaluée par un test immuno-enzymatique. Des séries de dilutions de plasma ont été incubées toute la nuit dans des plaques adsorbées avec Tat101, en présence ou en absence de Tat101 comme compétiteur (concentration finale de 2 µg/ml). Après trois cycles de lavage, 200 µl de réactif d'Ellman ont été ajoutés et l'absorbance à 414 nm a été mesurée après 1h.

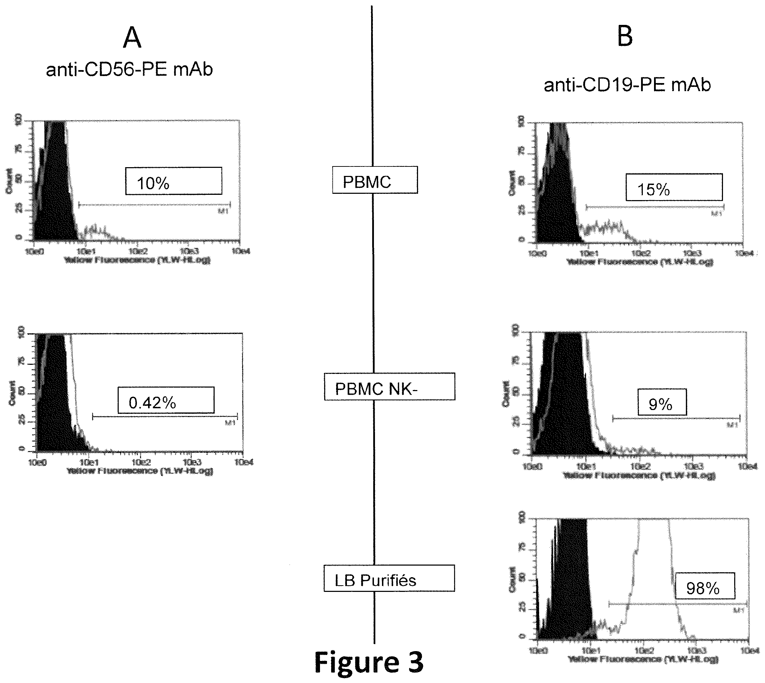

- la figure 3 montre la pureté de la population cellulaire révélée par cytométrie de flux. **A**. Les CMSP et la fraction de CMSP déplétées en cellules NK ont été incubées avec (histogramme gris) ou sans (histogramme noir) un anticorps anti-CD56 humain marqué à la phycoérythrine. **B.** Les CMSP, la fraction de CMSP déplétées en cellules NK et les lymphocytes B purifiés ont été incubés avec (histogramme gris) ou sans (histogramme noir) un anticorps anti-CD19 humain marqué à la phycoérythrine.

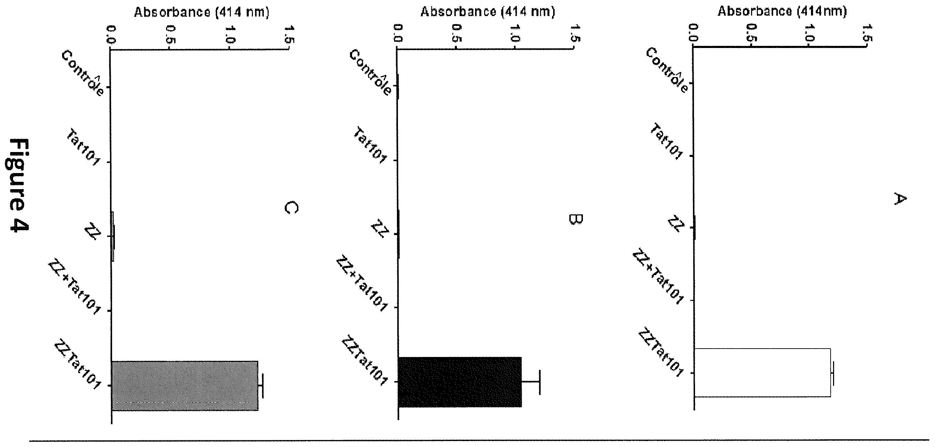

- la figure 4 montre l'effet de ZZTat101 sur la réponse en anticorps IgM. Réponse anticorps anti-Tat101 de CMSP-NK⁻ (1.10⁶ cellules/puits)(**A**), CMSP (1.10⁶ cellules/puits)(**B**), et de lymphocytes B purifiés (C) après immunisation avec la protéine de fusion ZZTat101 (concentration finale 10 µg/ml) ou ses composants : ZZ, Tat101, ou un mélange de ZZ et de Tat101 (concentration finale 10 µg/ml). Après une restimulation aux jours trois et sept de l'immunisation, la présence d'IgM spécifiques anti-Tat101 dans le surnageant de culture pur a été déterminée par un test immuno-enzymatique en présence ou en absence de Tat101 comme compétiteur (2 µg/ml). Après trois cycles de lavage, 200 µl de réactif d'Ellman ont été ajoutés et l'absorbance à 414 nm a été mesurée après 2h. Le signal spécifique correspond au signal en présence de Tat101 soustrait du signal en l'absence de Tat101.

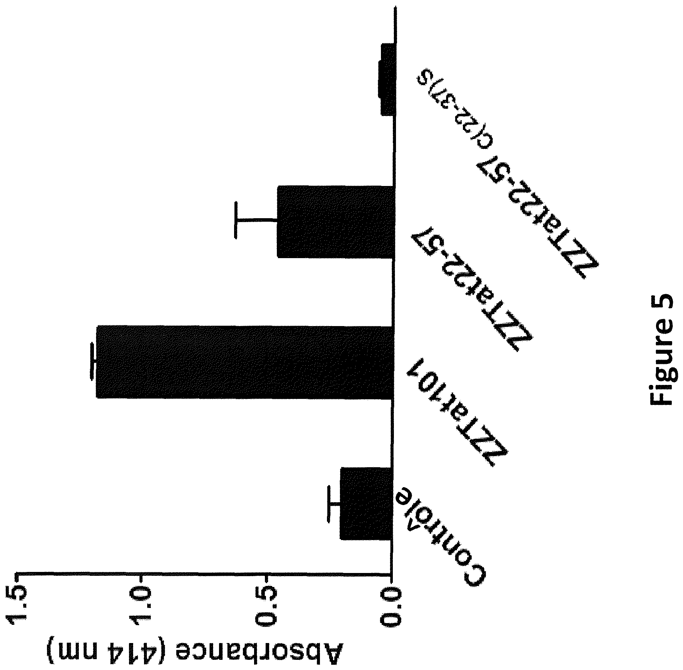

- la figure 5 montre que la région 22-57 de Tat101 est suffisante pour initier une réponse immune IgM, et que la réponse dépend de la présence des sept cystéines de Tat. Des CMSP déplétées en NK ont été incubées avec la protéine de fusion ZZTat101 ou ses dérivés, ZZTat22-57 incluant, les régions riche en cystéines (22-37), core (38-48) et basique (49-57), ZZ-Tat22-57_{C(22-37)S} correspondant à ZZTat22-57 avec les sept cystéines substituées en sérines. Les antigènes ont été ajoutés une seconde fois, à la même concentration, après 3 jours d'incubation. Les surnageants de culture ont été prélevés le septième jour et ont été utilisés pur pour déterminer la présence d'IgM spécifiques anti-Tat101 par un test immuno-enzymatique. Dans ce test, les surnageants ont été incubés dans des plaques en présence ou en absence d'un solution de Tat101 (2 µg/ml) qui a été utilisée comme compétiteur pour la liaison aux plaques. Après trois cycles de lavage, 200 µl de réactif d'Ellman ont été ajoutés et l'absorbance à 414 nm a été mesurée après 2h. Le signal spécifique correspond au signal en présence de Tat101 soustrait du signal en l'absence de Tat101.
- la figure 6 représente l'analyse par SDS-PAGE de la capacité de ZZ-Tat22-57 et ZZ-Tat22-57_{C(22-37)S} à former des oligomères. Dans cette expérience, ZZTat22-57 est dissous en tampon PBS (10µg de protéine dans 10µl de tampon) en présence d'un excès de maléimide pour bloquer les cystéines libres présentes dès la dissolution. Une autre solution de ZZTat22-57 est incubée pendant 6 jours à température ambiante en PBS. Après 6 jours, les cystéines libres résiduelles sont bloquées avec un excès de maléimide. Les deux échantillons de ZZTat22-57 (j=0 et j=6) ainsi que la protéine ZZTat22-57C(22-37)S dépourvue de cystéine sont ensuite déposées sur un gel SDS-PAGE en absence de réducteur pour évaluer la présence de formes monomériques et oligomériques.

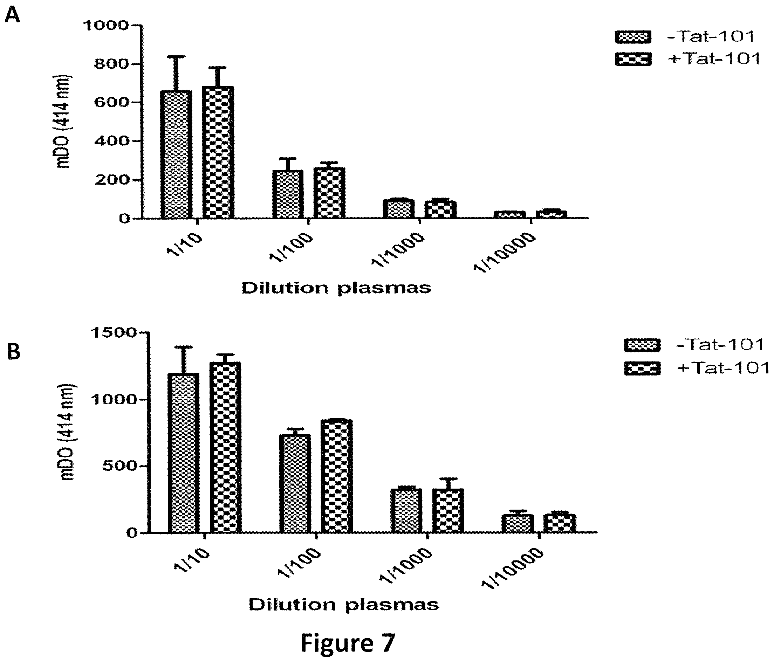

- la figure 7 montre l'analyse de la présence d'Acs IgM ou IgG anti-Tat dans les plasmas issus des résidus de concentré leucoplaquettaire. Pour évaluer la présence d'anticorps anti-Tat, des dilutions de plasma ont été incubées en présence ou en absence d'une solution de Tat101 dans des plaques de microtitration dont les puits ont préalablement adsorbés avec la protéine Tat. La présence d'IgM a été évaluée à l'aide d'un Ac anti-IgM couplé à la péroxydase (**A**) alors que la présence d'IgG a été évaluée à l'aide d'un Ac anti-IgG couplé à la péroxydase (**B**).

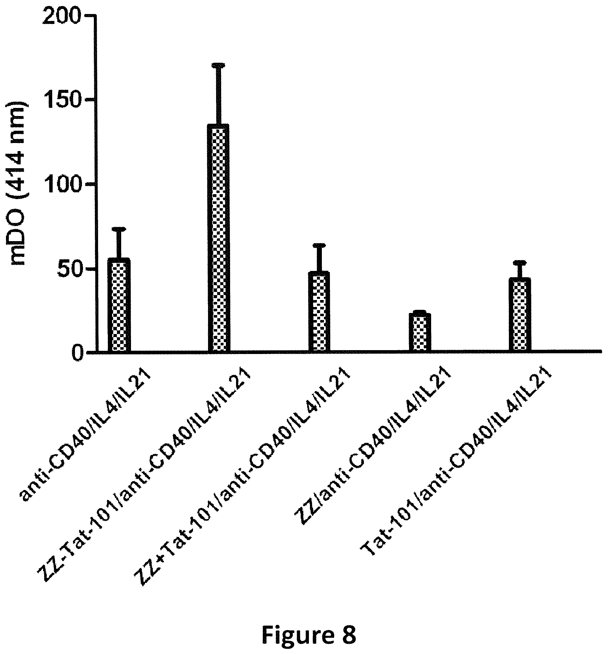

- la figure 8 montre que seuls les surnageants issus de l'incubation avec ZZ-Tat101 contiennent des IgG capables de lier les plaques adsorbées avec Tat101. Les CMSP ont été incubées avec un mélange d'activation (anti-CD40 à 1µg/ml, IL-4 à 10 ng/ml, IL-21 à 50 ng/ml) et en absence ou présence de différents Ags utilisés à 10µg/ml (ZZ-Tat101, ZZ, ZZ+Tat101, Tat101). Les surnageants ont ensuite été prélevés à différent temps et la présence d'anticorps a été évaluée par dosage immunoenzymatique en utilisant un anticorps de chèvre anti-IgG humaine couplé à la péroxydase.

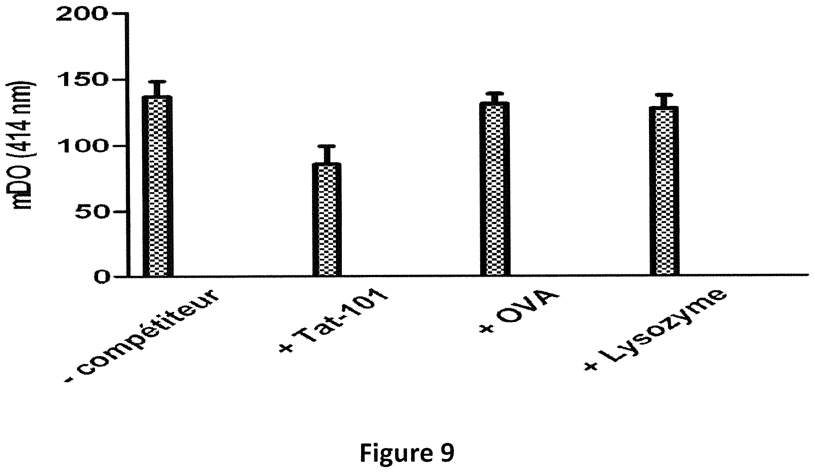

- la figure 9 montre que les Acs contenus dans les surnageants issus de l'incubation avec ZZ-Tat101 qui lient la plaque adsorbée avec Tat101 sont bien spécifiques de Tat. Pour déterminer la spécificité de liaison aux plaques, les surnageants incubés en absence d'Ag ou avec ZZ-Tat101 ont été incubés sur les plaques de microtitration en présence de solutions contenant soit la protéine Tat101, soit deux Ags non reliés (l'ovalbumine, appelée OVA, et le lysozyme). La liaison d'Acs aux plaques a été déterminée par dosage immunoenzymatique en utilisant un anticorps de chèvre anti-IgG humaine couplé à la péroxydase.

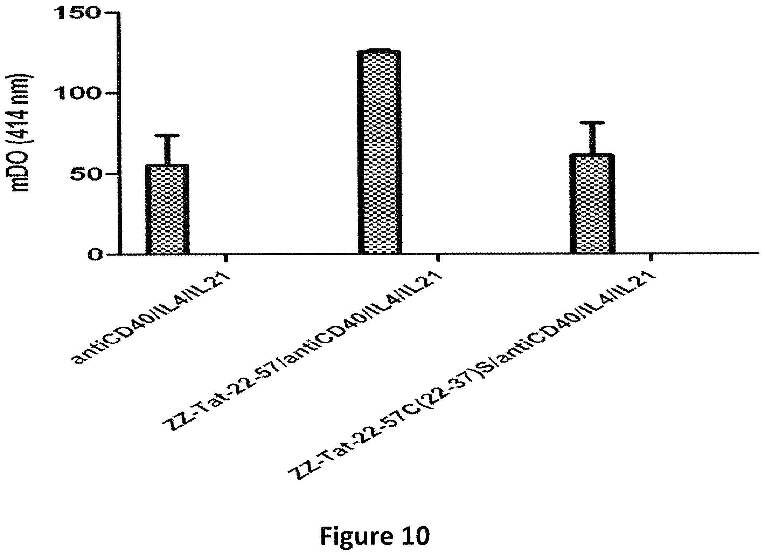

- la figure 10 montre que la région 22-57 de Tat suffit pour provoquer la sécrétion d'IgG et que la présence des cystéines localisées dans la région 22-37 est indispensable pour obtenir l'effet *in vitro.* Les CMSP ont été incubées avec un mélange d'activation (anti-CD40 à 1µg/ml, IL-4 à 10ng/ml, IL-21 à 50ng/ml) et en absence ou présence de ZZ-Tat22-57 ou de ZZ-Tat22-57_{C(22-37)S}. Les surnageants ont ensuite été prélevés à différent temps et la présence d'anticorps a été évaluée par dosage immunoenzymatique en utilisant un anticorps de chèvre anti-IgG humaine couplé à la péroxydase.

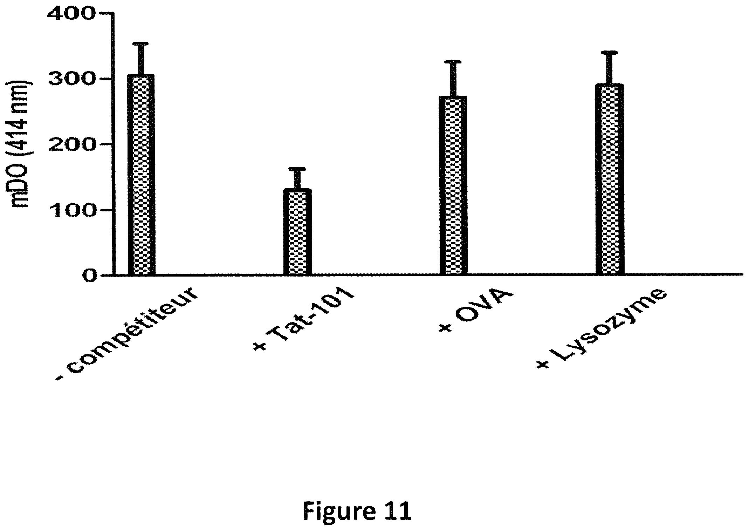

- la figure 11 montre que les Acs contenus dans les surnageants issus de l'incubation avec ZZ-Tat22-57 qui lient la plaque adsorbée avec Tat101 sont bien spécifiques de Tat. Pour déterminer la spécificité de liaison aux plaques, les surnageants incubés en absence d'Ag ou avec ZZ-Tat22-57 ont été incubés sur les plaques de microtitration en présence de solutions contenant soit la protéine Tat101, soit deux Ags non reliés (l'ovalbumine, appelée OVA, et le lysozyme). La liaison des Acs aux plaques a été déterminée par dosage immunoenzymatique en utilisant un anticorps de chèvre anti-IgG humaine couplé à la péroxydase.

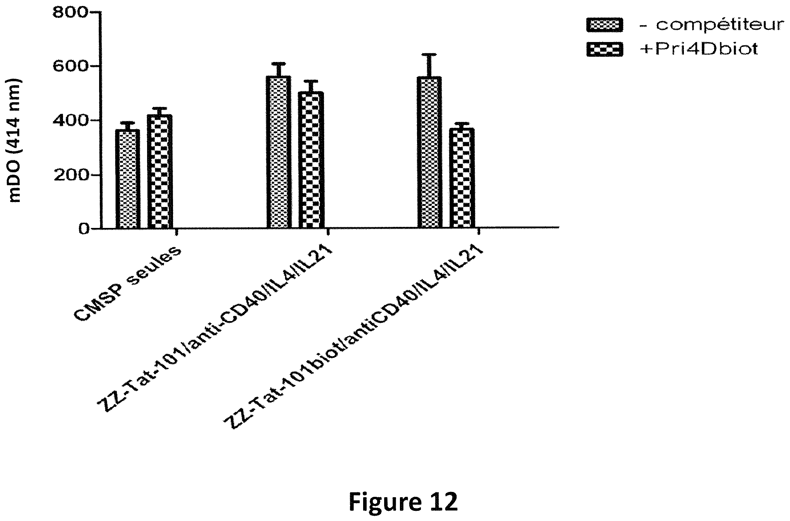

- la figure 12 montre que la biotine devient capable de déclencher « in vitro » une réponse en IgG quand elle est préalablement couplé à ZZ-Tat101. Les CMSP ont été incubées en présence ou en absence d'un mélange d'activation (anti-CD40 à 1µg/ml, IL-4 à 10ng/ml, IL-21 à 50ng/ml) et en absence ou présence de (ZZ-Tat101biot). Les surnageants ont été prélevés 11 jours plus tard. Des plaques de microtitration ont été adsorbées avec un peptide biotinylé, appelé Pri4Dbiot, dont la séquence peptidique n'est pas reliée à celle de ZZ-Tat101. Les surnageants ont ensuite été incubés sur les plaques en présence ou en absence d'une solution contenant un excès de peptide Pri4Dbiot. La présence d'anticorps a enfin été évaluée par dosage immunoenzymatique en utilisant un anticorps de chèvre anti-IgG humaine couplé à la péroxydase.

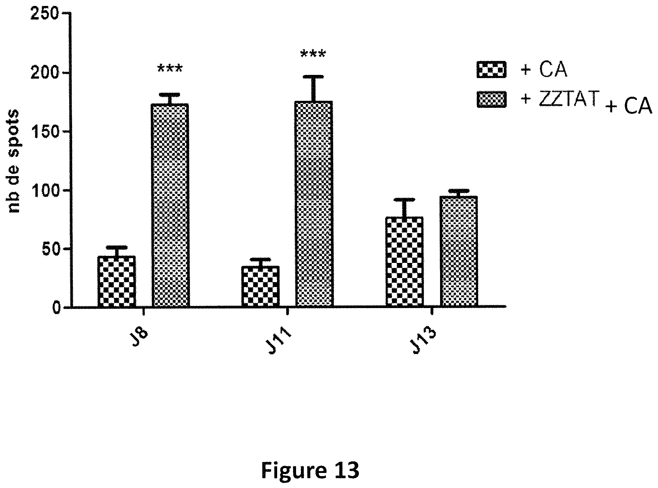

- la figure 13 montre qu'après immunisation *in vitro* les CMSPs contiennent des cellules sécrétrices d'IgG anti-Tat. Les CMSPs ont été incubées en présence d'un mélange d'activation (anti-CD40 à 1µg/ml, IL-4 à 10ng/ml, IL-21 à 50ng/ml) et en l'absence ou présence de ZZ-Tat101. Huit, onze ou treize jours plus tard, les cellules ont été transférées sur des plaques ELISPOT préalablement adsorbées avec Tat101. Après un jour d'incubation à 37°C, les plaques ont été lavées pour éliminer les cellules et un anticorps anti-IgG couplé à la péroxydase a été rajouté. Après 1 h d'incubation à 20°C, les plaques sont lavées et un mélange de BCIP/NBT est ajouté pour révéler l'activité enzymatique dans les puits. Les spots sont visualisés et comptés à l'aide d'un lecteur ELISpot.

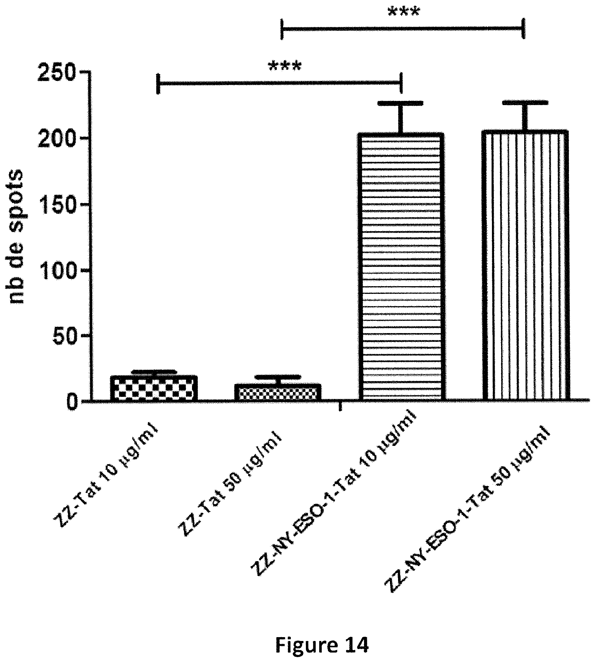

- la figure 14 montre qu'après immunisation *in vitro* les CMSPs contiennent des cellules sécrétrices d'IgG anti-NYESO-1. Les CMSPs ont été incubées en présence ou en absence d'un mélange d'activation (anti-CD40 à 1µg/ml, IL-4 à 10ng/ml, IL-21 à 50ng/ml) et en absence ou présence de ZZ-NY-ESO-1-Tat ou de ZZ-Tat. Onze jours plus tard, les cellules ont été transférées sur des plaques ELISPOT préalablement adsorbées avec le peptide NY-ESO-1. Après un jour d'incubation à 37°C, les plaques ont été lavées pour éliminer les cellules et un anticorps anti-IgG couplé à la péroxydase a été rajouté. Après 1h d'incubation à 20°C, les plaques ont été lavées et un mélange de BCIP/NBT ajouté pour révéler l'activité enzymatique dans les puits. Les spots ont été visualisés et comptés à l'aide d'un lecteur ELISpot. La figure présente le nombre de spots après déduction de ceux comptés lorsque les CMSPs sont incubées uniquement avec le cocktail d'activateurs.

### Exemple 1: Etude du pouvoir transactivateur de Tat101 et ZZ-Tat101.

### 1. Matériels et méthodes

### 1.1 Synthèse de la protéine Tat

La protéine Tat, dénommée Tat ou Tat101 (SEQ ID NO : 3) présente la séquence de l'isolat NDK du VIH-1 (Groenink et al., J Virol., 1991, 65, 1968-1975) qui correspond à une séquence consensus précédemment obtenue à partir de 66 séquences d'isolats primaires de VIH-1 rapportées dans les bases de données SWISSPROT et TrEMBL entre 1999 et 2000 (Kittiworakarn et al., J.Biol.Chem., 2006, 281 3105-3115). La synthèse chimique de Tat a été réalisée par la stratégie Fmoc/tert-butyl à l'aide d'un synthétiseur automatique de peptides APPLIED BIOSYSTEMS 433A. Le procédé chimique utilise 0,1 mmole de résine Fmoc-Asp(OtBu)-PAL-PEG-PS, un excès d'un facteur 10 de chaque acide aminé, du dicyclohexylcarbodiimide/1-hydroxy-7-azabenzotriazole et du diisopropyl-ethylamine/N-methyl pyrrolidone. La glutamine 54 a éventuellement été incorporée manuellement. Le clivage et la déprotection ont été réalisés à l'aide d'un mélange acide trifluoroacetique/triisopropylsilane/eau (9,5/0,25/0,25, v/v/v). Le matériel brut a été précipité deux fois avec du ter-butyle méthyle éther refroidi 4 °C puis dissous dans une solution aqueuse d'acide acétique à 15 %. La protéine brute a ensuite été purifiée par chromatographie liquide haute performance (CLHP) en phase inverse, sur une colonne Vydac® C18 (HESPERIA) ou une colonne JUPITER™ C4. Les groupements S(tBu) ont été enlevés des cystéines à l'aide de tampon phosphate 0,1M, pH 8,5 dégazé contenant 6M d'urée (6M) et du dithiothreitol (50 eq/Cys). Après avoir terminé la déprotection des cystéines, le mélange a été acidifié à pH 2,2 et purifié par CLHP sur une colonne C4. Les protéines Tat complètement réduites ont été conservées sous forme lyophilisée à -20°C. La protéine synthétisée a été caractérisée par spectrométrie de masse et par l'analyse des acides aminés.

### 1.2 Construction, expression et purification des protéines ZZ-Tat101 et ZZ-Tat22-57.

La protéine de fusion ZZTat101 a été construite en utilisant une séquence nucléotidique synthétique SEQ ID NO : 4 codant pour Tat101 flanquée par des sites *KpnI* et *BamHI,* respectivement en 5' et en 3'. La séquence nucléotidique a été insérée entre les sites *KpnI* et *BamHI* du vecteur pCP (Drevet et al., Protein Expression Purif. 1997, 10, 293-300) codant pour la protéine ZZ de séquence en acides aminés SEQ ID NO : 2, un double domaine de liaison à la région Fc des IgGs dérivé du domaine B de la protéine A de *Staphylococcus aureus,* précédemment décrit (Nilsson et al., Protein Engineering 1987, 1,107-113).

La protéine de fusion ZZTat22-57 a été construite de façon similaire en utilisant une séquence nucléotidique synthétique SEQ ID NO : 5 codant pour le peptide 22-57 de Tat101, flanquée par des sites *KpnI* et *BamHI,* respectivement en 5' et en 3'.

La protéine de fusion ZZTat22-57_{C(22-37)S} contient Tat22-57_{C(22-37)S}, un variant de Tat22-57 dans lequel toutes les cystéines de la région riche en cystéines (C22, 25, 27, 30, 31, 34 et 37) ont été substituées par des sérines, fusionné à ZZ. La protéine de fusion ZZTat22-57_{C(22-37)S} a été construite de façon similaire en utilisant une séquence nucléotidique synthétique SEQ ID NO : 6 codant pour Tat22-57_{C(22-37)S}, flanquée par des sites *KpnI* et *BamHI,* respectivement en 5' et en 3'.

Des bactéries (*E. coli* BL21(DE3)pLysS) ont ensuite été transformées avec les différents plasmides. Les protéines de fusion ont été exprimées puis purifiées sur une colonne d'affinité (IgG Sepharose™6 Fast flow, AMERSHAM). La pureté a été évaluée par gel d'électrophorèse. Les protéines ont été produites avec des rendements variant entre 1 à 5 mg/l de culture. Les produits ont été conservés sous forme lyophylisée jusqu'à utilisation.

### 1.3 Evaluation de l'activité de transactivation de Tat101 et ZZ-Tat101

L'activité de transactivation a été évaluée en utilisant le protocole décrit par Kittiworakarn et al. (J. Biol. Chem., 2006, 281, 3105-15).

### 2. Résultats

La capacité de Tat à déclencher la réponse immunitaire a été étudiée en utilisant une protéine Tat de 101 résidus, appelée Tat101, qui provient d'un isolat viral décrit précédemment (Groenink et al., J Virol., 1991, 65, 1968-1975). Comme l'activité de transactivation de Tat peut causer la dérégulation de nombreux gènes (Li et al., AIDS, 2010, 1609-23 ; Darbinian-Sarkissian et al., J. Cell Physiol., 2006, 208, 506-15) et avoir des effets activateurs ou inhibiteurs, le pouvoir transactivateur de Tat101 et ZZTat101 a été évalué. Le protocole utilisé est fondé sur l'incubation des protéines en présence de cellules HeLa transfectées avec un plasmide codant pour la séquence LTR d'HIV-1 et pour la séquence de la GFP (Kittiworakarn et al., J. Biol. Chem., 2006, 281, 3105-15). Comme on peut le voir sur la figure 1, la protéine Tat101 sauvage transactive efficacement puisqu'elle augmente l'expression de la GFP par la lignée HeLa. En revanche, ZZTat101 ne provoque pas d'augmentation de l'expression de la GFP ce qui indique que la protéine de fusion est dépourvue de l'activité de transactivation de Tat et qu'elle ne peut donc déréguler des gènes par l'intermédiaire de cette activité.

### Exemple 2 : L'incubation in vitro de CMSP en présence de ZZ-Tat101 ou de ZZ-Tat22-57 provoque la sécrétion d'IgM spécifiques de Tat

### 1.Matériels et méthodes

### 1.1 Purification des cellules mononuclées du sang humain périphérique

Le sang (environ 250 ml) de donneurs sains (négatifs pour HIV1/2, HTLV-I/II, HCV et HBsAg) provient de l'Etablissement Français du Sang (France). Les CMSP sont isolées par deux centrifugations successives en gradient de densité (Histopaque®-1077, SIGMA) à 1200g pendant 30 min. Les plasmas des donneurs sont isolés et conservés à -80°C pour l'analyse sérologique. Les cellules sont rincées en PBS (10 mM phosphate de potassium pH7,4 et 150 mM NaCl) additionné de 2 mM EDTA.

### 1.2 Séparation cellulaire

### Isolement des lymphocytes B

Les lymphocytes B sont isolés à partir des CMSP en utilisant des microbilles MACS® selon le protocole du fabricant (MILTENYI BIOTEC). Brièvement, 5.10⁸ CMSP sont lavées dans du tampon MACS (PBS additionné de 2 mM EDTA et 0,5 % BSA), à 4°C. Des microbilles anti-CD20 (150 µl) sont ajoutées (5 µl de microbilles anti-CD20 pour 10⁷ cellules) et les cellules sont incubées à 4°C pendant 15 min. Les cellules sont rincées, centrifugées, et passées au travers de colonnes magnétiques. L'enrichissement est évalué par analyse en cytométrie de flux (Guava®, MILLIPORE), en utilisant un anticorps anti-CD19 humain conjugué à la phycoérythrine (MILTENYI BIOTEC).

### Déplétion des cellules NK (Natural Killer)

Les cellules NK sont déplétées des CMSP à l'aide de microbilles MACS® selon le protocole du fabricant (MILTENYI BIOTEC). Brièvement, 1.10⁸ CMSP sont lavées dans du tampon MACS (PBS additionné de 2 mM EDTA et 0,5 % BSA), à 4°C. Des microbilles anti-CD56 (800 µl) sont ajoutées (80 µl de microbilles anti-CD56 pour 10⁷ cellules) et les cellules sont incubées à 4°C pendant 15 min. Les cellules sont rincées, centrifugées, et passées au travers de colonnes magnétiques. L'enrichissement est évalué par analyse en cytométrie de flux (Guava®, MILLIPORE) en utilisant un anticorps anti-CD56 humain conjugué à la phycoérythrine ((MILTENYI BIOTEC).

Les CMSP, lymphocytes B purifiés et CMSP déplétées en NK sont cultivées à une densité de 1.106 cellules/ml dans du milieu RPMI-1640 supplémenté en L-glutamine (2 mM), pénicilline (50 UI/ml), streptomycine (50 µg/ml), 50 µM beta-mercaptoethanol et 10 % de sérum de veau foetal inactivé par la chaleur.

### 1.3 Analyse en cytométrie de flux

La cytométrie de flux a été utilisée pour analyser différents marqueurs de la membrane cellulaire et évaluer ainsi la qualité de la séparation cellulaire. Toutes les procédures de marquage en cytométrie de flux ont été réalisées à 4°C dans du PBS additionné de 1 % de BSA. 10⁵ cellules de chaque sous-population cellulaire (CMSP, lymphocytes B purifiés et CMSP déplétées en NK) sont marquées pendant 35 min avec un anticorps anti-CD19 humain conjugué à la phycoérythrine (MILTENYI BIOTEC) et avec un anticorps anti-CD56 humain conjugué à la fluorescéine (BD BIOSCIENCES), puis lavées. Ensuite, les cellules sont fixées avec du paraformaldéhyde 4 % pendant 10 min à température ambiante et lavées avant l'analyse en FACS, à l'aide d'un cytomètre de flux à trois couleurs (Guava®).

### 1.4 Préparation des plaques pour test immunoenzymatique (ELISA)

Des plaques à 96 puits (MAXISORP™, NUNC) ont été recouvertes de peptides Tat (P1 (1-15), P10(46-60), P15(66-80), P16(71-85), P18 (86-101)), Tat101, et BSA (0.1 µg/puits en tampon phosphate de sodium 50 mM, pH 7,4), pendant une nuit à température ambiante. Les plaques ont été lavées une fois avec du tampon de lavage (phosphate de potassium 0,01 M, pH7,4 contenant 0,05 % de Tween 20) et saturées avec du tampon pour test immunoenzymatique (EIA) (phosphate 0,1M, pH 7,4 contenant 0,15 M NaCl , 0,1 % BSA et 0,01 % azide de sodium) et conservées à 4°C. Avant utilisation, les plaque sont lavées trois fois avec du tampon de lavage.

### 1.5 Préparation de l'anticorps conjugué

Les anticorps monoclonaux (mAbs) sont liés à l'acétylcholinestérase (AChE) en utilisant un protocole décrit précédemment (Grassi et al., J. Immunol. Methods, 1989, 123, 193-210). Brièvement, des fragments F(ab')₂ sont obtenus à partir d'anticorps purifiés par traitement à la pepsine en milieu acide. Des fragments Fab' sont obtenus à partir des F(ab')₂ par réduction en présence de 2-mercaptoethyl-amine 0,02M puis couplés à l'AChE en utilisant le récatif hétérobifonctionnel N-succinimidyl-4-(N-maleimidomethyl)-cyclohexane-1-caboxylate (SMCC), comme décrit précédemment (Grassi et al., J. Immunol. Methods, 1989, 123, 193-210).

### 1.6 Mesure de l'activité acétylcholinestérase

L'activité AChE a été mesurée par la méthode d'Ellman (Ellman et al., Biochem. Pharmacol., 1961, 7, 88-95). Le milieu d'Ellman comprend un mélange d'acétylthiocholine 7,5.10⁻⁴ (substrat enzymatique) et de 5,5'-dithiobis(2-nitrobenzoïque acide) 2,5.10⁻⁴ M (DTNB ; réactif pour la mesure colorimétrique des thiols), dans un tampon phosphate 0,1 M, pH 7,4. L'activité enzymatique est exprimée en unités d'Ellman (EU). Une EU est définie comme la quantité d'enzyme produisant une augmentation d'absorbance d'une unité pendant 1 min dans 1 ml de milieu, pour un chemin optique de 1 cm et correspond à environ 8 ng d'enzyme.

### 1.7 Détection des anticorps anti-Tat

La présence d'anticorps anti-Tat a été analysée dans les plasmas des donneurs de sang et dans les surnageants de culture cellulaire d'immunisation *in vitro.* 50 µl de séries de dilutions de plasma ou de surnageants de puits d'immunisation *in vitro* ont été transférés dans des microplaques recouvertes de Tat, de peptides de Tat et de BSA avec 50 µl de tampon EIA ou 50 µl de Tat101 (2 µg/ml dans tampon EIA) pour déterminer la spécificité. Après une nuit à 4°C, les plaques ont été lavées trois fois avec du tampon de lavage avant de révéler la présence d'anticorps anti-Tat. Pour détecter les IgM anti-Tat, 50 µl d'anticorps de lapin anti-IgM humaines (1 µg/ml, JACKSON IMMUNORESEARCH) ont été ajoutés aux plaques, qui ont été incubées toute la nuit à 4°C. Après lavage, 50 µl de 2,5 EU/ml d'anticorps de souris anti-immunoglobulines de lapin couplé à l'AChE ont été ajoutés pendant 4 h à température ambiante. Pour détecter les IgG anti-Tat, 50 µl d'anticorps de souris anti-IgG humaines couplé à l'AChE (anticorps monoclonal de MERIDIAN LIFE SCIENCE ; 2,5 EU/ml) ont été ajoutés et incubés toute la nuit à 4°C. Après trois cycles de lavages, 200 µl de réactif d'Ellman ont été ajoutés et l'absorbance a été mesurée à 414 nm, après 1h.

### 1.8 Immunisation in vitro

L'immunisation in vitro des CMSP, CMSP déplétées en NK et lymphocytes B purifiés a été réalisée dans les plaques à 24 puits, à 1.106 cellules par puits dans un volume final de 1 ml de milieu (RPMI-1640 supplémenté en L-glutamine (2 mM), pénicilline (50 UI/ml), streptomycine (50 µg/ml), 50 µM beta-mercaptoethanol et 10 % de sérum de veau foetal inactivé par la chaleur). Les différentes populations cellulaires ont été incubées *in vitro,* en présence ou en absence de respectivement, ZZTat101, ZZ, Tat101, et un mélange contenant ZZ+Tat101. Les puits de culture ont été incubés avec 50 µl d'une solution de 200 µg/ml de chaque antigène. A J7, les surnageants des puits ont été testés par ELISA pour détecter les anticorps spécifiques produits par les cellules. Dans une deuxième série d'expériences, les cellules ont été incubées *in vitro* avec 10 µg/puits de différentes protéines de fusion apparentées à ZZTat101, *i.e.,* ZZTat22-57_{C(22-37)S} et ZZTat22-57. Les expériences ont ensuite été réalisées comme les précédentes.

### 2. Résultats

### 2.1 Sélection d'échantillons sanguins dépourvu d'anticorps anti-Tat

Dans la mesure où la présente étude a pour but d'évaluer la capacité de Tat101 à induire une réponse immune *in vitro* avec des lymphocytes B naïfs pour cet antigène, des échantillons de sang ont été préalablement sélectionnés, en utilisant comme critère l'absence d'anticorps anti-Tat101 dans leurs plasmas. Pour évaluer la présence d'anticorps anti-Tat101, des séries de dilutions de différents échantillons de plasmas ont été incubées sur des microplaques adsorbées avec Tat101, ou avec de la SAB, en présence ou en absence d'une solution contenant un excès de Tat101. L'interaction entre les anticorps et la protéine adsorbée sur la microplaque a été révélée à l'aide d'anticorps anti-IgG et anti-IgM marqués à l'AChE. Après soustraction du signal observé sur les plaques contrôle adsorbées avec la SAB à celui mesuré sur les plaques adsorbées avec Tat101, aucune différence n'a été observée entre les signaux obtenus en présence ou en l'absence de Tat101 comme compétiteur, quelque soit la dilution de plasma utilisée (Figure 2). Ces résultats démontrent l'absence d'IgM (Figure 2A) ou d'IgG (Figure 2B) anti-Tat101 dans les plasmas et suggère que le sang correspondant contient des cellules B naïves pour Tat.

### 2.2 Préparation de différentes sous-populations cellulaires pour l'immunisation in vitro

La capacité de Tat101 à induire la production d'anticorps a été examinée en utilisant trois populations cellulaires différentes. Les CMSP incluent toutes les cellules mononuclées retrouvées *in vivo.* Les CMSP déplétées en NK sont dépourvues des cellules précédemment décrites comme inhibant la réponse immunitaire *in vitro.* Enfin, des lymphocytes B purifiés ont été utilisés dans le but d'étudier l'aptitude de Tat101 à induire la production d'anticorps en l'absence de cellules dendritiques et de lymphocytes T auxiliaires. Les CMSP déplétées en NK ont été récupérées à partir des CMSP comme des cellules non-retenues après purification des cellules NK à l'aide d'anticorps anti-CD56 couplés à des billes magnétiques. L'analyse en cytométrie de flux (Figure 3A) a montré que les cellules NK représentaient environ 10 % des CMSP avant fractionnement, alors que 0,42 % au plus des cellules NK subsistaient après chromatographie d'affinité, démontrant un rendement de plus de 95 % pour cette étape de déplétion. Une procédure opposée a été utilisée pour purifier les lymphocytes B. L'étape de chromatographie d'affinité implique des anticorps anti-CD20 couplés à des billes magnétiques pour retenir et purifier spécifiquement les lymphocytes B. L'analyse en cytométrie de flux (Figure 3B) a montré que les lymphocytes B représentaient environ 15 % des CMSP, alors que le pourcentage de lymphocytes B atteint 98 % après purification sur des billes magnétiques.

### 2.3 Expériences d'immunisation in vitro avec Tat101 et ZZTat101

Afin de comparer l'aptitude de Tat101 libre, ou fusionnée à ZZ à déclencher la production d'anticorps spécifiques in vitro, Tat101, ZZ, ZZTat101 et un mélange de ZZ et Tat101 ont été comparés dans les expériences d'immunisation *in vitro.* Des études préliminaires réalisées en présence ou en l'absence d'Il-2, d'IL-4 et d'un ligand du TLR-9, CpG ODN 2006 (5'-TCGTCGTTTTGTCGTTTTGTCGTT-3' ; SEQ ID NO : 7) ont montré que le cocktail cytokines/CpG ne conférait aucun bénéfice à la réponse immunitaire anti-Tat101. Par conséquent, toutes les expériences ont été réalisées sans cytokines et sans CpG.

Après 7 jours d'incubation à 37°C des différentes populations cellulaires avec les différents antigènes, les surnageants ont été récoltés et analysés en utilisant un ELISA pour détecter les IgM et les IgG spécifiques de Tat101. L'analyse de la présence d'IgM antiTat101 dans les surnageants issus de CMSP déplétées en NK (figure 4A) montre une absence de signal pour les surnageants issus de l'incubation avec la protéine Tat101 libre ou de l'incubation contrôle réalisée en absence d'antigène. Les mêmes résultats ont été observés lorsque la réponse IgG a été analysée. Ces résultats démontrent que la protéine Tat101 n'est pas capable d'initier une réponse anticorps *in vitro.* Ce comportement change lorsque Tat101 est fusionnée à ZZ, étant donné qu'un signal IgM est observé dans les surnageants résultant de l'incubation avec ZZTat101. Ce signal spécifique n'a pas été observé en utilisant Tat101 mélangé à ZZ ou ZZ seul, ce qui montre que ni ZZ seul, ni un mélange des deux protéines libres n'est capable de déclencher une réponse IgM spécifique.

L'ensemble de ces résultats montre que seule l'association covalente de Tat101 et ZZ est capable de déclencher une réponse humorale spécifique *in vitro.* Le fait qu'aucune réponse ne soit déclenchée lorsque Tat libre est incubée en présence de ZZ indique que l'effet obtenu n'est pas lié à des mécanismes « bystander » qui pourraient provenir du domaine de liaison des IgG. De plus le phénomène n'est pas relié à l'activité biologique principale de Tat, *i.e.,* son activité de transactivation puisque ZZTat101 est dépourvue d'activité transactivatrice (Figure 1).

La sécrétion d'anticorps a été observée en l'absence de cytokines qui sont habituellement utilisées dans des expériences d'immunisation *in vitro.* Cependant, alors que des IgMs anti-Tat ont systématiquement été observées, la présence d'IgG spécifiques n'a été trouvée que dans une expérience sur six. Cela suggère que ZZTat101, à elle seule, fourni les signaux nécessaires pour déclencher une réponse immunitaire primaire mais n'est pas capable d'induire la commutation isotypique.

Des résultats similaires ont été observés lorsque la fraction totale de CMSP a été utilisée (Figure 4B), indiquant que les cellules NK ne modifient pas la stimulation *in vitro* fournie par ZZTat101. Par conséquent, l'utilisation de ZZTat101 permet de s'affranchir de l'étape de déplétion en cellules immunosuppressives habituellement utilisée dans les protocoles d'immunisation *in vitro,* et donc de simplifier les procédures d'immunisation *in vitro* sans diminuer l'efficacité de l'immunisation *in vitro.*

Le même comportement a également été observé en utilisant des lymphocytes B purifiés (Figure 4C), suggérant que la stimulation *in vitro* induite par ZZTat101 ne nécessite pas la présence de cellules dendritiques et de lymphocytes T auxiliaires.

De plus, le système génétique utilisé pour produire ZZTat101, qui permet la production de plusieurs antigènes en tandem, est un outil utile pour exprimer d'autres protéines liées à Tat, de façon à fournir aux antigènes incorporés une capacité de stimulation *in vitro.*

### 2.4 Détermination du déterminant moléculaire de la protéine de fusion impliqué dans la réponse immune humorale in vitro.

Une série de dérivés de ZZTat101 ont été préparés pour identifier le déterminant moléculaire de la protéine de fusion impliqué dans l'induction d'une réponse immune humorale *in vitro.* Le premier, appelé ZZTat22-57, comprend la séquence 22-57 de Tat et le second, appelé ZZTat22-57_{C(22-37)S}, correspond à un mutant de ZZTat22-57 dans lequel les sept cystéines de la région riche en cystéines (résidus 22 à 37) ont été remplacées par des sérines. ZZTat101, ZZTat22-57 et ZZTat22-57_{C(22-37)S} ont été incubés en présence de CMSP déplétées en cellules NK pour comparer leur capacité à déclencher la réponse immune humorale *in vitro.* Après 7 jours d'incubation, les surnageants ont été récupérés et ajoutés dans des puits de microplaques préalablement adsorbés avec Tat101. Par comparaison avec les surnageants de l'incubation avec ZZTat101, les surnageants de l'incubation avec ZZTat22-57 montrent une production réduite d'IgM. Toutefois, cette production est supérieure à celle observée avec les surnageants contrôle (figure 5), ce qui indique que la région 22-57 de Tat déclenche une réponse immune significative, *in vitro.* Par opposition, aucune réponse n'a été observée avec les surnageants issus de l'incubation avec ZZTat22-57_{C(22-37)S} qui a un domaine ZZ intact mais est dépourvue des sept cystéines de Tat, ce qui indique que dans la protéine de fusion ZZTat101, c'est la protéine Tat qui joue un rôle crucial dans l'induction de la réponse immune humorale *in vitro.* Ces résultats indiquent également qu'une ou plusieurs des cystéines de Tat contribuent à ce phénomène. La capacité de ZZTat22-57 à déclencher une réponse immune *in vitro* indique que le déterminant moléculaire est principalement situé dans la région 22-57. De façon intéressante, la région riche en cystéines située entre les résidus 22 et 37 est elle-même située à l'intérieur du déterminant moléculaire contrôlant l'effet.

L'analyse de ZZTat22-57_{C(22-37)}, après séparation en SDS-PAGE (figure 6), montre que contrairement à ZZTat22-57 qui est capable d'oligomériser, ZZTat22-57_{C(22-37)S} est incapable d'oligomériser. Ces résultats indiquent que la capacité de ZZTat101 à induire une réponse immune humorale *in vitro* est liée aux propriétés d'oligomérisation de Tat, médiées par ces résidus de cystéines.

### Exemple 3 : L'incubation in vitro de CMSP en présence de ZZ-Tat101 ou de ZZ-Tat22-57 provoque la sécrétion d'IgG spécifiques de Tat.

### 1. Matériels et méthodes

### 1.1 Immunisation in vitro

Les résidus de concentré leuco-plaquétaire de donneurs sains (négatifs pour HIV1/2, HTLV-I/II, HCV et HBsAg) provenant de l'Etablissement Français du Sang (France) sont déposés sur ficoll (Histopaque®-1077, SIGMA) et les CMSP sont isolés par une centrifugation en gradient de densité à 1200g pendant 15 min. Une lyse osmotique des hématies est ensuite réalisée avec 10 ml de tampon de lyse (8.3g NH4Cl, 0.84g NaHCO3, 0.5 ml EDTA 0.2M, qsp IL H₂O). Après 10 minutes d'incubation à 4°C, les cellules sont lavées avec 50 ml PBS additionné de 2 mM EDTA.

Les cellules sont diluées en milieu RPMI-1640 additionné de 2mM L-glutamine, pénicilline (50 UI/ml), streptomycine (50 µg/ml), 50 µM β-mercapto-éthanol et 10 % de sérum de veau foetal inactivé par la chaleur) et déposées dans des plaques 96 puits à raison de 5.10⁵ cellules par puits. Les puits contiennent aussi l'Ag (ZZ-Tat101, ZZ ou ZZ + Tat101) à 10µg/ml, un anticorps antiCD40 (1 µg/ml), de l'IL-4 (10 ng/ml), et de l'IL-21 (50 ng/ml). Les mélanges sont incubés à 37°C et les surnageants sont prélevés à 11 jours plus tard pour évaluer la présence d'anticorps spécifiques de Tat101.

### 1.2 Dosage des Acs dans les surnageants de culture

La présence d'IgG spécifiques de Tat dans les surnageants de culture est évaluée par dosage immunoenzymatique. Des plaques de microtitration sont adsorbées avec la protéine Tat (100µl/pts à 1µg/ml en tampon phosphate 0.1M pH7,4). Les puits sont ensuite saturés avec une solution de tampon phosphate 0.1M pH7,4 contenant 0,3 % sérum-albumine bovine (SAB, 200µl/puits). Après une nuit à 4°C, les plaques sont lavées et les surnageants dilués au 1/4 sont ajoutés dans les puits (50µl/pts) en présence ou en absence de différents compétiteurs. Après une nuit à 4°C, les puits sont lavés et un anticorps anti-IgG humain couplé à la péroxydase est ajouté au puits. Après 30 minutes d'incubation les plaques sont lavées et du 2,2'-azino-bis[3-ethylbenzothiazoline-6-acide sulphonique] (ABTS) est ajouté pour révéler l'activité enzymatique dans les puits.

### 2 Résultats

Pour déterminer la capacité de Tat101 libre ou couplé à ZZ, à induire la réponse immunitaire *in vitro,* il était indispensable d'utiliser des CMSP naïves pour cet antigène. Cette sélection a été effectuée en se basant sur l'absence d'Ac anti-Tat dans les plasmas issus des résidus de concentré leucoplaquettaire contenant les CMSP. La présence d'Acs a été évaluée en utilisant des plaques de microtitration préalablement adsorbées avec Tat101 ou avec de la SAB. Ces plaques ont été incubées avec des séries de dilution des plasmas, en présence ou en absence d'une solution de Tat101 (10µg/ml). L'interaction des Acs avec les plaques a ensuite été mesurée par dosage immunoenzymatique à l'aide d'Ac antiIgG et antiIgM. Comme on peut le voir sur la figure 7A qui représente la liaison des IgM aux plaques Tat101, une liaison est mesurable pour des dilutions de plasma de 1/10, 1/100 et 1/1000. Cependant, cette liaison n'est pas diminuée en présence de l'excès de Tat101 en solution ce qui indique qu'elle n'est pas spécifique de la protéine. Le même comportement est observé pour la mesure de la liaison des IgG (figure 7B). Ces résultats indiquent donc que les plasmas testés sont dépourvus d'anticorps IgG et IgM anti-Tat et indiquent que les poches de sang contiennent des cellules B naïves pour Tat.

Les résidus de concentré leucoplaquettaire ont ensuite été traités à l'aide d'un ficoll pour récupérer les CMSP. La capacité de Tat101 et ZZ-Tat101 à déclencher la réponse immunitaire *in vitro* a été déterminée en utilisant des CMSP un anticorps antiCD40 et deux cytokines (IL-4 et IL-21) qui contribuent à la réponse immunitaire humorale ainsi qu'au switch isotypique. Les CMSP et le mélange cytokinique ont été incubés en absence ou en présence de Tat101, ZZ-Tat101, ZZ, ZZ+ Tat, respectivement. Après différents temps d'incubation à 37°C, les surnageants ont été prélevés et évalués pour la présence d'IgG spécifiques de Tat. Comme on peut le voir sur la figure 8, les surnageants issus de l'incubation avec ZZ-Tat101 contiennent des IgG capables de lier les puits de microtitration qui ont été préalablement adsorbés avec la protéine Tat101. En revanche, le signal optique obtenu avec les surnageants issus de l'incubation avec Tat101, ZZ ou ZZ + Tat101 ne diffère pas significativement de celui mesuré pour les surnageants issus de l'incubation des CMSP en absence d'Ag. Ces données suggèrent donc que seuls les surnageants issus de l'incubation avec ZZ-Tat101 contiennent des IgG spécifiques de Tat. Pour déterminer si la liaison aux plaques est réellement spécifique de Tat, l'inhibition de cette liaison a ensuite été évaluée en présence de solutions contenant soit la protéine Tat101, soit deux Ags non reliés (l'ovalbumine, appelée OVA, et le lysozyme). Comme on peut le voir sur la figure 9, la liaison n'est pas significativement modifiée en présence d'OVA ou de lysozyme alors qu'elle est complètement abolie en présence de la solution de Tat101 ce qui démontre que les anticorps détectés sont spécifiques de Tat. L'ensemble de ces résultats indique donc que la protéine de fusion ZZ-Tat101 est capable de déclencher une réponse immunitaire humorale *in vitro* et que l'effet nécessite le couplage covalent de ZZ et Tat101.

Pour délimiter le déterminant moléculaire impliqué dans la réponse immunitaire humorale *in vitro,* deux mutants de ZZ-Tat ont été préparés. Le premier, appelé ZZTat22-57, a été choisi car il comporte la région 22-57 de Tat qui contient les domaines riche en cytéines (résidus 22 à 37), core (résidus 38 à 48) et basique (49 à 57) qui sont impliquées dans de nombreuses activités biologiques de Tat. Le second correspond à un mutant de ZZTat22-57 dans lequel les 7 résidus cystéine ont été remplacés par des sérines. ZZTat22-57 et ZZTat22-57_{C(22-37)S} ont été incubés en présence de CMSP afin de comparer leur aptitude à déclencher la réponse immunitaire *in vitro.* Après 11 jours d'incubation, les surnageants ont été recueillis et ajoutés à des puits de microtitration préalablement adsorbés avec Tat101. Comme on peut le voir sur la figure 10, le surnageant de ZZTat22-57 présente une liaison aux plaques supérieure à la liaison du surnageant issu de l'incubation avec le cocktail d'activation contrôle (antiCD40/IL-4/IL-21) ce qui indique que la protéine de fusion incluant la région 22-57 de Tat est capable de déclencher la réponse immunitaire *in vitro.* En revanche, les surnageants issus de l'incubation avec ZZTat22-57_{C(22-37)S} ne se lient pas plus efficacement aux plaques que les surnageants issus de l'incubation avec le cocktail d'activation contrôle (antiCD40/IL-4/IL-21). Comme on peut le voir sur la figure 11, la réponse immunitaire induite par ZZTat22-57 est spécifique de Tat car deux solutions contenant respectivement de l'ovalbumine (OVA) et du lysozyme sont incapables d'inhiber la liaison des anticorps aux plaques alors que la solution contenant Tat-101 diminue significativement la liaison des IgG. La capacité à déclencher la réponse immunitaire *in vitro* dépend de l'aptitude à former des oligomères médiés par les cystéines car contrairement à ZZTat22-27 qui est capable d'oligomériser, ZZTat22-57_{C(22-37)S} est incapable d'oligomériser (figure 6).

### Exemple 4 : La capacité à déclencher la réponse immunitaire in vitro peut être conférée à un autre Ag par couplage covalent à ZZ-Tat101

### 1. Matériels et méthodes

### 1.1. Couplage de la biotine à ZZ-Tat101

La protéine ZZ-Tat101 (1mg) est dissoute dans 700µl de tampon phosphate 0,1M pH7,5. La biotine réactive (2mg de biotinamidocaproate N-hydroxy-succinimide ester) est dissoute dans 100µl de diméthylformamide. Les solutions sont mélangées et incubées pendant une heure à température ambiante sous agitation. La réaction est arrétée par ajout de glycine (200µl dilué à 0,1M en tampon phosphate 0,1M pH7,5). Le mélange réactionnel est ensuite conservé à -20°C sous forme lyophylisée. Le couplage de la biotine à ZZ-Tat101 est évalué par un test de dosage immunoenzymatique. Pour réaliser ce test, des plaques de microtitration sont tout d'abord adsorbées par incubation une nuit à 4°C avec une solution d'IgG de lapin (100µg/puits à 1µg/ml en tampon phosphate 0,05M pH7,2). Les plaques sont saturées par incubation pendant une nuit à 4°C avec une solution de tampon phosphate 0,1M pH7,2 contenant 0,3% de SAB (200µl/puits). Les plaques sont ensuite lavées et des dilutions du mélange réactionnel sont ajoutées. Après une heure d'incubation à température ambiante, les plaques sont lavées et la liaison de ZZ-Tat101biot aux IgG adsorbées est révélée en utilisant de la streptavidine couplée à la péroxydase et de l'ABTS comme substrat.

### 1.2. Immunisation in vitro

Les expériences d'immunisation *in vitro* sont réalisées en utilisant un protocole similaire à celui décrit dans l'exemple 2. Les CMSP et le mélange d'activation sont incubées en présence ou en absence de ZZTat101-biot. Après 11jours à 37°C, les surnageants sont prélevés pour évaluer la présence d'anticorps spécifiques de la biotine.

### 1.3 Dosage des Acs dans les surnageants de culture.

La présence d'IgG spécifiques de la biotine dans les surnageants de culture est évaluée par dosage immunoenzymatique. Des plaques de microtitration sont adsorbées avec un peptide (100µl/pts à 1µg/ml en tampon phosphate 0.1M pH7,4), appelé Pri4Dbiot, qui est non relié à Tat et couplé à la biotine. Les puits sont ensuite saturés avec une solution de tampon phosphate 0.1M pH7,4 contenant 0,3% sérum-albumine bovine (200µl/puits). Après une nuit à 4°C, les plaques sont lavées et les surnageants dilués au 1/4 sont ajoutés dans les puits (50µl/pts) en présence ou en absence de différents compétiteurs. Après une nuit à 4°C, les puits sont lavées et un anticorps antiIgG humain couplé à la péroxydase est ajouté aux puits. Après 30 minutes d'incubation les plaques sont lavées et de l'ABTS est ajouté pour révéler l'activité enzymatique dans les puits.

### 2. Résultats

Pour évaluer si la capacité de ZZ-Tat à déclencher une réponse en IgG *in vitro* peut être transférée à d'autres antigènes, la biotine a été choisie étant donné qu'il s'agit d'un haptène incapable de déclencher seul la réponse immunitaire. La biotine a été couplée chimiquement à la protéine de fusion ZZ-Tat101 afin de produire un composé ZZ-Tat-Ag, appelé ZZ-Tat101-biot. Des CMSP ont ensuite été incubés dans les conditions de culture utilisées précédemment pour ZZ-Tat101 en présence ou en absence de ZZ-Tat101-biot. Après 11 jours d'incubation à 37°C, les surnageants ont été prélevés afin d'évaluer la présence d'IgG anti-biotine par dosage immunoenzymatique. Un signal a été mesuré pour les surnageants issus de CMSP incubées sans Ag (figure 12). Ce signal n'est cependant pas inhibé par une solution contenant un excès de peptide Pri4D-biotine ce qui indique qu'il correspond à une liaison non spécifique aux plaques. Pour les surnageants issus de l'incubation avec ZZ-Tat101, un signal plus élevé a été mesuré. Il n'est cependant pas significativement inhibé en présence de la solution de Pri4Dbiot ce qui indique que la liaison aux plaques est non-spécifique. Pour les surnageants issus de l'incubation avec ZZ-Tat101-biot, un signal plus élevé que celui observé pour les surnageants issus de CMSP incubées sans Ag a été mesuré. Ce signal est de plus inhibé en présence de la solution de Pri4D-biotine ce qui indique qu'il correspond à la liaison d'IgG humaines spécifiques de la biotine. L'ensemble de ces résultats démontre donc que la capacité à déclencher une réponse humorale et la production d'IgG humaines spécifiques est conférée à la biotine quand elle est préalablement couplée à ZZ-Tat101.

### Exemple 5 : L'immunisation in vitro permet l'induction de lymphocytes B sécréteurs d'IgG spécifiques de l'Ag inclus dans la protéine de fusion.

### 1. Matériels et méthodes

### 1.1 Synthèse du peptide NY-ESO-1.

Un fragment de la protéine NY-ESO-1 (SLLMWITQCFLPVARGPESRLLEFYLAMPFATPMEAELARRSLA ; SEQ: ID NO : 8) a été synthétisé chimiquement par la stratégie Fmoc/tert-butyl à l'aide d'un synthétiseur automatique de peptides APPLIED BIOSYSTEMS 433A. Le procédé chimique utilise 0,1 mmole de résine Fmoc-Asp(OtBu)-PAL-PEG-PS, un excès d'un facteur 10 de chaque acide aminé, du dicyclohexylcarbodiimide/1-hydroxy-7-azabenzotriazole et du diisopropylethylamine/N-methyl pyrrolidone. Le clivage et la déprotection ont été réalisés à l'aide d'un mélange acide trifluoro-acetique/triisopropylsilane/eau (9,5/0,25/0,25, v/v/v). Le matériel brut a été précipité deux fois avec du ter-butyle méthyle éther refroidi 4 °C puis dissous dans une solution aqueuse d'acide acétique à 15 %. La protéine a ensuite été purifiée par chromatographie liquide haute performance (CLHP) en phase inverse, sur une colonne Vydac C18 (HESPERIA). Les peptides et les protéines synthétisés ont été caractérisés par spectrométrie de masse et par l'analyse des acides aminés. Ils sont conservés à -20°C, sous forme lyophilisée.

### 1.2 Construction, expression et purification de la protéine ZZ-NY-ESO-1-Tat

La protéine de fusion ZZ-NY-ESO-1-Tat contient le fragment de NY-ESO-1 et la région 22-57 de Tat101 fusionnés à ZZ. La séquence nucléotidique synthétique SEQ ID NO : 5 codant pour le peptide 22-57 de Tat 101 est décrite à l'exemple 1. Le peptide NY-ESO-1 est codé par la séquence nucléotidique : CTG CTG ATG TGG ATT ACC CAG TGC TTT CTG CCG GTG GCT CGT GGC CCG GAA AGC CGT CTG CTG GAA TTT TAC CTG GCG ATG CCG TTT GCG ACC CCG ATG GAA GCG GAA CTG GCG CGT CGT AGC CTG GCG (SEQ ID NO : 9). Ces deux séquences ont été insérées dans un vecteur pCP codant pour la protéine ZZ (Drevet et al., Protein Expression Purif., 1997, 10, 293-300), en utilisant les sites de restriction *SacI*/*KpnI*/*BamHI.*

Des bactéries (*E. coli* BL21de3) ont ensuite été transformées avec le plasmide. La protéine de fusion a été exprimée puis purifiée en utilisant une colonne d'affinité sur laquelle sont greffés des anticorps (Acs). La pureté a été évaluée par gel d'électrophorèse. La protéine a été produite avec des rendements variant entre 1 à 5 mg/l de culture. Elle a été conservée sous forme lyophylisée jusqu'à utilisation.

### 1.3 Immunisation in vitro

L'immunisation *in vitro* a été réalisée dans des plaques 96 puits par l'incubation de 5.10⁵ CMSPs par puits en présence d'un anticorps antiCD40 à 1 µg/ml, d'IL4 (10 ng/ml) d'IL21 (50 ng/ml) et en présence ou en l'absence de l'Ag ZZTat101 ou de l'Ag ZZ-NY-ESO-1Tat (concentration de 10µg/ml pour chaque protéine de fusion). Les mélanges ont été incubés à 37 °C pendant 8, 11 ou 13 jours puis la présence d'IgG spécifiques de Tat a été évaluée par un dosage ELISpot.

### 1.4. Dosage ELISpot

La production d'IgG spécifiques de Tat ou du peptide NY-ESO-1 par les lymphocytes B immunisés *in vitro* a été évaluée par dosage ELISpot. Des plaques de dosage ELISpot (Multiscreen™ MAHA, MILLIPORE™) ont été adsorbées avec la protéine Tat ou avec le peptide NY-ESO-1 (50 µl/puits à 1µg/ml en tampon carbonate 50 mM pH 9,6). Les puits ont ensuite été saturés avec du RPMI-1640 contenant 10 % de sérum de veau foetal inactivé par la chaleur (200 µl/puits). Après 2 h à 37°C, les plaques ont été lavées et les CMSP immunisées *in vitro* ont été déposées (100µl de chaque puits d'immunisation *in vitro*/puits de plaque ELISpot). Après 24 h d'incubation à 37 °C, les plaques ont été lavées et un anticorps anti-IgG humain couplé à la biotine a été ajouté au puits. Après 1h 30 min d'incubation à 20°C, les plaques ont été lavées et de la streptavidine couplée à la phosphatase alcaline a été ajoutée au puits. Après 1 h d'incubation à 20°C, les plaques ont été lavées et un mélange de BCIP (5-bromo-4-chloro-3-indolyl-phosphate)/ NBT (nitro blue tetrazolium ; PROMEGA) est ajouté pour révéler l'activité enzymatique dans les puits. Les spots sont visualisés et comptés sur un lecteur ELISpot.

### 2. Résultats

Pour confirmer que la méthode d'immunisation *in vitro* de l'invention permet l'induction de lymphocytes B sécréteurs d'IgG spécifiques de l'Ag inclus dans protéine de fusion, des CMSPs ont été incubées dans des plaques de culture avec le cocktail cytokinique d'activation et en présence ou l'absence de ZZTat101. Le mélange a ensuite été transféré dans des plaques ELISpot préalablement adsorbées avec Tat. Après 24 heures d'incubation à 37°C, les plaques ont été lavées pour éliminer les cellules et la présence de spots correspondant aux lymphocytes B capables de sécréter des IgG spécifiques de Tat a été révélée. Comme on peut le voir sur la figure 13 montrant le nombre de spots spécifiques de Tat en fonction du temps d'incubation des CMSPs en présence ou en l'absence de ZZTat, des spots sont mesurés dans les puits contenant les CMSPs et le cocktail activateur uniquement. Cependant, après 8 et 11 jours d'incubation, ce nombre de spots est significativement plus faible que celui mesuré dans les puits qui contenaient aussi ZZTat. Le comportement change après 13 jours d'incubation. En effet, le nombre de spots n'est plus significativement différent quand les CMSPs sont incubés avec le cocktail activateur, en l'absence ou en présence de ZZTat. L'ensemble de ces données indique donc que l'incubation de la protéine de fusion permet l'induction de lymphocytes B capables de sécréter des IgG anti-Tat avec un optimal de réponse qui se situe entre le jour 8 et le jour 11.

Pour confirmer que la méthode d'immunisation *in vitro* de l'invention permet l'induction de lymphocytes B sécréteurs d'IgG spécifiques d'un autre Ag que Tat, une protéine de fusion contenant un fragment de la protéine NY-ESO-1, appelée ZZ-NY-ESO-1-Tat, a été utilisée Des CMSPs ont été incubées dans des plaques de culture avec le cocktail cytokinique d'activation et en présence ou en l'absence de deux concentrations différentes de ZZ-NY-ESO-1-Tat ou de ZZ-Tat. Le mélange a ensuite été transféré dans des plaques ELISpot préalablement adsorbées avec le peptide NY-ESO-1. Après 24 heures d'incubation à 37°C, les plaques ont été lavées pour éliminer les cellules et la présence de spots correspondant aux lymphocytes B capables de sécréter des IgG spécifiques de NY-ESO-1 a été révélée. Comme on peut le voir sur la figure 14, un faible nombre de spots est compté quand les CMSPs sont incubées avec ZZ-Tat. Ce nombre de spots est environ cinq fois moins élevé que celui mesuré quand les CMSPs sont incubées avec ZZ-NY-ESO-1-Tat. Cette différence significative est retrouvée pour les deux concentrations de protéine qui ont été utilisées. L'ensemble de ces données indique donc que l'incubation de ZZ-NY-ESO-1-Tat avec les CMSPs permet l'induction de lymphocytes B capables de sécréter des IgG anti-NY-ESO-1.

### Exemple 6 : La fusion entre les CMSPs induites par immunisation in vitro et les cellules d'un hétéromyélome humain/murin conduit à l'obtention d'hybridomes B capables de sécréter des IgG spécifiques de Tat

### 1. Matériels et méthodes

### 1.1. Immunisation in vitro des CMSPs avant l'étape de fusion cellulaire

L'immunisation *in vitro* des CMSPs a été réalisée dans des boites de Pétri à raison de 25.10⁶ cellules par boite dans un volume final de 15 ml de milieu (RPMI-1640 supplémenté en L-glutamine (2 mM), pyruvate de sodium (1 mM), pénicilline (50 UI/ml), streptomycine (50 µg/ml) et 10 % de sérum de veau foetal inactivé par la chaleur). Les différentes populations cellulaires ont été incubées *in vitro* en présence de l'Ag ZZTat101 à 10 µg/ml, d'un anticorps antiCD40 à 1 µg/ml, d'IL4 à 10 ng/ml et d'IL21 à 50ng/ml. Les mélanges ont été incubés à 37°C pendant 11 jours puis les cellules ont été isolées en vue de l'étape de fusion cellulaire.

### 1.2. Culture des cellules de l'hétéromyélome humain/murin (HM) avant l'étape de fusion cellulaire

Les cellules de l'hétéromyélome humain/murin sont cultivées dans du milieu RPMI-1640 additionné de 2 mM L-glutamine, 1 mM pyruvate de sodium, pénicilline (50 UI/ml), streptomycine (50 µg/ml) et 10% de sérum de veau foetal inactivé par la chaleur.

### 1.3. Fusion cellulaire

Les CMSPs immunisées *in vitro* (4.10⁷ cellules) ont été mélangées aux cellules d'hétéromyélome (2.10⁷ cellules) dans du milieu RPMI-1640 puis le mélange cellulaire est centrifugé (10 minutes à 1000 rpm). La fusion cellulaire a été réalisée grâce à l'ajout goutte à goutte de l'agent fusionnant polyéthylène glycol 50% (PEG 400, SIGMA ALDRICH). Les cellules ont ensuite été lavées, reprises en milieu sélectif (RPMI-1640 additionné de 2 mM L-glutamine, 1 mM pyruvate de sodium, 1 % acides aminés, pénicilline (50 UI/ml), streptomycine (50 µg/ml), 20 % de sérum de veau foetal inactivé par la chaleur) supplémenté avec de l'hypoxantine (0.1 mM), de l'aminoptérine (0.4 mM) et de la thymidine (16 mM) puis déposées dans des plaques 96 puits (100 µL/puits).

### 1.4. Sélection des hybridomes produisant des IgGs spécifiques de Tat

Des plaques de dosage immunoenzymatique ont été préparées. Dans ces plaques, la protéine Tat (100µl/puits à 1µg/ml en tampon phosphate 0.1M pH7,4) a été incubée pendant une nuit à 4°C dans les plaques de microtitration. Les puits ont ensuite été saturés avec une solution de tampon phosphate 0.1M pH7,4 contenant 0,3 % sérum-albumine bovine (SAB, 200µl/puits). Après une nuit à 4°C, les plaques ont été lavées et les surnageants de cultures issus de chaque puits contenant les cellules fusionnées ont été dilués au 1/10 et ajoutés à ces plaques (100 µl/puits). Après une nuit à 4°C, les plaques ont été lavées et un anticorps anti-IgG humain couplé à la péroxydase a été ajouté aux puits. Après 30 minutes d'incubation, les plaques ont été lavées et du 2,2'-azino-bis[3-ethylbenzothiazoline-6-acide sulphonique] (ABTS) a été ajouté pour révéler l'activité enzymatique dans les puits. La valeur seuil utilisée pour identifier les puits contenant des hybridomes sécréteurs d'IgG spécifiques de Tat correspond à la densité optique moyenne des huit puits contenant uniquement HM plus trois écarts-type (l'écart-type est défini à partir des signaux obtenus pour les huit puits contenant uniquement HM).

### 2. Résultats

Des expériences de fusion cellulaire avec un hétéromyélome humain/murin (HM) ont été réalisées pour évaluer si les CMSPs induites par immunisation *in vitro* peuvent être immortalisées et produire des IgG spécifiques de l'Ag inclus dans la protéine de fusion. Dans ces expériences, des CMSPs induites par immunisation *in vitro* avec ZZTat101 ont été incubées en présence d'HM et de PEG400. A la suite de cette fusion, les cellules ont été réparties dans des puits de plaque de microtitration en présence d'un milieu sélectif permettant d'éliminer l'hétéromyélome non fusionné. Les surnageants contenus dans les puits ont ensuite été prélevés et la présence d'IgG spécifiques de Tat a été mesurée par dosage immuno-enzymatique. Les résultats sont présentés dans les Tableaux I et II.

**Tableau I : Dosages ELISA des IgGs anti-Tat dans les surnageants d'hybridomes**

| | | | | | | | | | | | | HM seul |
|---|---|---|---|---|---|---|---|---|---|---|---|---|
| **TAT 1** | **1** | **2** | **3** | **4** | **5** | **6** | **7** | **8** | **9** | **10** | **11** | **12** |
| **A** | 0,058 | 0,039 | 0,036 | 0,052 | 0,042 | 0,042 | 0,045 | 0,037 | 0,051 | 0,048 | 0,05 | 0,091 |
| **B** | 0,022 | 0,01 | 0,018 | 0,024 | 0,032 | 0,079 | 0,071 | 0,065 | 0,062 | 0,053 | 0,028 | 0,056 |
| **C** | 0,022 | 0,015 | 0,043 | 0,065 | 0,055 | 0,123 | 0,165 | 0,183 | 0,116 | 0,035 | 0,031 | 0,041 |
| **D** | 0,017 | 0,028 | 0,047 | 0,086 | 0,165 | 0,1 | 0,083 | 0,079 | 0,035 | 0,024 | 0,037 | 0,031 |
| **E** | 0,012 | 0,012 | 0,017 | 0,042 | 0,054 | 0,055 | 0,027 | 0,014 | 0,012 | 0,019 | 0,015 | 0,021 |
| **F** | 0,017 | 0,007 | 0,026 | 0,009 | 0,006 | 0,008 | 0,011 | 0,011 | 0,011 | 0,011 | 0,01 | 0,023 |
| **G** | 0,03 | 0,012 | 0,015 | 0,008 | 0,006 | 0,009 | 0,008 | 0,003 | 0,01 | 0,007 | 0,012 | 0,04 |
| **H** | 0,058 | 0,036 | 0,031 | 0,032 | 0,044 | 0,034 | 0,033 | 0,022 | 0,044 | 0,034 | 0,047 | 0,063 |

**Tableau II: Dosages ELISA des IgGs anti-Tat dans les surnageants d'hybridomes**

| **TAT 3** | **1** | **2** | **3** | **4** | **5** | **6** | **7** | **8** | **9** | **10** | **11** | **12** |
|---|---|---|---|---|---|---|---|---|---|---|---|---|
| **A** | 0,032 | 0,023 | 0,013 | 0,022 | 0,059 | 0,019 | 0,021 | 0,02 | 0,032 | 0,038 | 0,036 | 0,052 |
| **B** | 0,033 | 0,007 | 0,002 | 0,004 | 0,008 | 0,011 | 0,032 | 0,047 | 0,06 | 0,037 | 0,004 | 0,031 |
| **C** | 0,015 | 0,017 | 0,059 | 0,041 | 0,097 | 0,115 | 0,136 | 0,092 | 0,048 | 0,098 | 0,036 | 0,031 |
| **D** | 0,013 | 0,135 | 0,043 | 0,111 | 0,199 | 0,088 | 0,178 | 0,185 | 0,174 | 0,127 | 0,036 | 0,026 |
| **E** | 0,016 | 0,105 | 0,064 | 0,091 | 0,145 | 0,114 | 0,065 | 0,162 | 0,128 | 0,056 | 0,009 | 0,019 |
| **F** | 0,021 | 0 | -0,005 | -0,003 | -0,006 | 0,006 | 0,011 | 0,017 | 0,007 | 0,029 | -0,002 | 0,018 |
| **G** | 0,027 | 0,001 | -0,003 | -0,002 | -0,005 | -0,005 | -0,004 | -0,003 | -0,004 | 0,021 | 0,001 | 0,019 |
| **H** | 0,049 | 0,028 | 0,02 | 0,023 | 0,016 | 0,021 | 0,024 | 0,023 | 0,023 | 0,021 | 0,034 | 0,044 |

Comme on peut le voir sur les Tableaux I et II, cinq puits sont positifs dans la plaque nommée Tat1, alors que dix puits sont positifs dans la plaque nommée Tat3. Ces données indiquent donc que la fusion entre les CMSPs induites par immunisation *in vitro* avec ZZTat101 et les cellules d'un hétéromyélome humain/murin conduit à l'obtention de 15 hybridomes B capables de sécréter des IgG spécifiques de Tat.

## Revendications

1. Méthode d'immunisation *in vitro* de lymphocytes B humains, comprenant la culture d'une population totale de cellules mononuclées du sang humain périphérique en présence d'une composition antigénique comprenant au moins un antigène lié de façon covalente à la fois à :
(i) une protéine Tat ou à un fragment de Tat capables de s'oligomériser par l'intermédiaire de cystéine(s) de la région riche en cystéines de Tat et
(ii) un ligand d'une molécule de surface spécifique des cellules présentatrices d'antigène,
le fragment de Tat capable de s'oligomériser comprenant les régions riche en cystéines, core et basique, et
le ligand de ladite molécule de surface spécifique des cellules présentatrices d'antigène étant sélectionné dans le groupe comprenant : la protéine BB, la protéine ZZ, un anticorps anti-CMH de classe II, anti-RFcgamma, anti-IgM, anti-IgD, anti-IgG, anti-DEC-205, anti-CD209 ou anti-CD20 et un fragment des anticorps précédents comprenant au moins le paratope.

2. Méthode d'immunisation *in vitro* selon la revendication 1, dans laquelle ledit fragment est choisi parmi le peptide Tat 22-57 et les variants dudit peptide possédant une à six des cystéines C22, C25, C27, C30, C31, C34 et C37 en référence à la séquence SEQ ID NO : 3, les cystéines restantes de la séquence du peptide étant substituées par un autre acide aminé.

3. Méthode d'immunisation *in vitro* selon l'une quelconque des revendications 1 ou 2, dans laquelle ladite composition antigénique comprend au moins l'antigène lié de façon covalente à une protéine de fusion entre la protéine ZZ et une protéine Tat ou un fragment de Tat choisi parmi le peptide Tat 22-57 et les variants dudit peptide possédant une à six des cystéines C22, C25, C27, C30, C31, C34 et C37 en référence à la séquence SEQ ID NO : 3, les cystéines restantes de la séquence du peptide étant substituées par un autre acide aminé.

4. Méthode d'immunisation *in vitro* selon la revendication 3, dans laquelle ladite composition comprend également un anticorps anti-CMH de classe II, un anticorps anti-RFcgamma, anti-IgD, anti-IgM, anti-IgG, anti-DEC-205, anti-CD209 ou anti-CD20, lié de façon non-covalente à ladite protéine ZZ.

5. Méthode d'immunisation *in vitro* selon l'une quelconque des revendications 1 à 4, dans laquelle ledit antigène est une cible pour le diagnostic ou le traitement d'une maladie.

6. Méthode d'immunisation *in vitro* selon la revendication 5, dans laquelle ladite maladie est choisie parmi les cancers, les maladies auto-immunes, les maladies causées par des agents pathogènes et leurs toxines, les maladies inflammatoires chroniques, et le rejet de greffe.

7. Méthode d'immunisation *in vitro* selon l'une quelconque des revendications 1 à 6, dans laquelle ladite composition antigénique comprend au moins un facteur d'activation des lymphocytes B et/ou un facteur de différenciation des lymphocytes B.

8. Méthode de production d'anticorps humains spécifiques d'un antigène, comprenant :
a) l'immunisation *in vitro* de lymphocytes B humains avec un antigène selon la méthode d'immunisation *in vitro* des revendications 1 à 7,
b) l'immortalisation des lymphocytes B immunisés obtenus à l'étape a), et
c) la récupération des anticorps humains spécifiques de l'antigène produits par les lymphocytes B immortalisés obtenus à l'étape b).

9. Méthode de production d'anticorps selon la revendication 8, comprenant une étape supplémentaire de clonage des lymphocytes B immortalisés, entre les étapes b) et c).

10. Méthode de production d'anticorps selon la revendication 8 ou 9, dans laquelle lesdits anticorps spécifiques de l'antigène sont des anticorps humains d'isotype IgG.

11. Méthode de production d'anticorps selon l'une quelconque des revendications 8 à 10, dans laquelle lesdits anticorps spécifiques de l'antigène sont des anticorps monoclonaux humains.

## Patentansprüche

1. Verfahren zur In-vitro-Immunisierung von menschlichen B-Lymphozyten, welches die Kultur einer Gesamtpopulation mononukleärer Zellen des menschlichen peripheren Bluts in Gegenwart einer antigenen Zusammensetzung umfasst, die wenigstens ein Antigen umfasst, das gleichzeitig kovalent gebunden ist an:
(i) ein TAT-Protein oder ein TAT-Fragment, das zur Oligomerisierung mittels Cystein(en) aus der Cystein-reichen TAT-Region fähig ist, und
(ii) einen Liganden eines Moleküls mit spezifischer Oberfläche der Antigen-präsentierenden Zellen,
wobei das oligomerisierbare TAT-Fragment Cystein-reiche Kern- und Basisbereiche umfasst, und
wobei der Ligand des Moleküls mit spezifischer Oberfläche der Antigen-präsentierenden Zellen gewählt ist aus der Gruppe, umfassend: BB-Protein, ZZ-Protein, Anti-CMH-Antikörper der Klasse II, anti-RFcgamma, anti-IgM, anti-IgD, anti-IgG, anti-DEC-205, anti-CD209 oder anti-CD20 und ein Fragment der vorhergehenden Antikörper, welches wenigstens das Paratop aufweist.

2. Verfahren zur In-vitro-Immunisierung nach Anspruch 1, wobei das Fragment gewählt ist aus dem TAT 22-57-Peptid und den Varianten des Peptids, welche ein bis sechs der Cysteine C22, C25, C27, C30, C31, C34 und C37 aufweisen, mit Bezug auf die Sequenz SEQ ID NR: 3, wobei die verbleibenden Cysteine der Peptidsequenz durch eine andere Aminosäure substituiert werden.

3. Verfahren zur In-vitro-Immunisierung nach einem der Ansprüche 1 oder 2, wobei die antigene Zusammensetzung wenigstens das kovalent an ein Fusionsprotein gebundene Antigen umfasst, zwischen dem ZZ-Protein und einem TAT-Protein oder einem TAT-Fragment, gewählt aus dem TAT 22-57-Peptid und den Varianten des Peptids, welche ein bis sechs der Cysteine C22, C25, C27, C30, C31, C34 und C37 mit Bezug auf die Sequenz SEQ ID NR: 3 aufweisen, wobei die verbleibenden Cysteine der Peptidsequenz durch eine andere Aminosäure substituiert werden.

4. Verfahren zur In-vitro-Immunisierung nach Anspruch 3, wobei die Zusammensetzung auch einen anti-CMH-Antikörper der Klasse II, einen anti-RFcgamma, anti-IgD, anti-IgM, anti-DEC-205, anti-CD209 oder anti-CD20 Antikörper umfasst, nicht-kovalent an das ZZ-Protein gebunden.

5. Verfahren zur In-vitro-Immunisierung nach einem der Ansprüche 1 bis 4, wobei das Antigen ein Ziel für die Diagnostik oder Behandlung einer Krankheit ist.

6. Verfahren zur In-vitro-Immunisierung nach Anspruch 5, wobei die Krankheit gewählt ist aus Krebserkrankungen, Autoimmunerkrankungen, Krankheiten, die durch Krankheitserreger und deren Toxine verursacht werden, chronisch-entzündliche Erkrankungen und Transplantatabstoßung.

7. Verfahren zur In-vitro-Immunisierung nach einem der Ansprüche 1 bis 6, wobei die antigene Zusammensetzung wenigstens einen B-Lymphozyten-Aktivierungsfaktor und/oder einen B-Lymphozyten-Differenzierungsfaktor umfasst.

8. Verfahren zur Herstellung von menschlichen Antikörpern, die für ein Antigen spezifisch sind, umfassend:
a) In-vitro-Immunisierung von menschlichen B-Lymphozyten mit einem Antigen nach dem In-vitro-Immunisierungsverfahren der Ansprüche 1 bis 7,
b) Immortalisierung der in Schritt a) erhaltenen immunisierten B-Lymphozyten, und
c) Wiedergewinnung der spezifischen menschlichen Antikörper des durch die in Schritt b) erhaltenen immortalisierten B-Lymphozyten produzierten Antigens.

9. Verfahren zur Herstellung von Antikörpern nach Anspruch 8, umfassend einen zusätzlichen Schritt des Klonens der immortalisierten B-Lymphozyten zwischen den Schritten b) und c).

10. Verfahren zur Herstellung von Antikörpern nach Anspruch 8 oder 9, wobei die spezifischen Antikörper des Antigens menschliche Antikörper vom IgG-Isotyp sind.

11. Verfahren zur Herstellung von Antikörpern nach einem der Ansprüche 8 bis 10, wobei die spezifischen Antikörper des Antigens menschliche monoklonale Antikörper sind.

## Claims

1. Method for the *in vitro* immunisation of human B lymphocytes, comprising culturing a total population of human peripheral blood mononuclear cells in the presence of an antigenic composition comprising at least one antigen covalently bonded to both:
(i) a Tat protein or a Tat fragment capable of oligomerisation via one or more cysteines of the cysteine-rich region of Tat and
(ii) a ligand of a surface molecule specific to antigen presenting cells,
the Tat fragment capable of oligomerisation comprising the cysteine-rich, core and basic regions, and
the ligand of said surface molecule specific to antigen presenting cells being selected from the group consisting of: the BB protein, ZZ protein, anti-MHC Class II antibody, anti-FcyR antibody, anti-IgM antibody, anti-IgD antibody, anti-IgG antibody, anti-DEC-205 antibody, anti-CD209 antibody or anti-CD20 antibody, and a fragment of the preceding antibodies comprising at least the paratope.

2. Method for *in vitro* immunisation according to claim 1, wherein said fragment is chosen from the group consisting of: the Tat(22-57) peptide and variants of said peptide having one to six of the cysteines C22, C25, C27, C30, C31, C34 and C37 with reference to the sequence SEQ ID NO: 3, the remaining cysteines of the sequence of the peptide being substituted by another amino acid.

3. Method for *in vitro* immunisation according to either claim 1 or claim 2, wherein said antigenic composition comprises at least the antigen covalently bonded to a fusion protein between the ZZ protein and a Tat protein or a Tat fragment selected from the group consisting of the Tat(22-57) peptide and the variants of said peptide having one to six of the cysteines C22, C25, C27, C30, C31, C34 and C37 with reference to the sequence SEQ ID NO: 3, the remaining cysteines of the sequence of the peptide being substituted by another amino acid.

4. Method for *in vitro* immunisation according to claim 3, wherein said composition further comprises an anti-MHC Class II antibody, an anti-FcyR antibody, an anti-IgD antibody, an anti-IgM antibody, an anti-IgG antibody, an anti-DEC-205 antibody, an anti-CD209 antibody or an anti-CD20 antibody, non-covalently bonded to said ZZ protein.

5. Method for *in vitro* immunisation according to any of claims 1 to 4, wherein said antigen is a target for the diagnosis or treatment of a disease.

6. Method for *in vitro* immunisation according to claim 5, wherein said disease is selected from the group consisting of: cancers, auto-immune diseases, diseases caused by pathogenic agents and the toxins thereof, chronic inflammatory diseases, and graft rejection.

7. Method for *in vitro* immunisation according to any of claims 1 to 6, wherein said antigenic composition comprises at least one B lymphocyte activating factor and/or one B lymphocyte differentiation factor.

8. Method for producing antigen-specific human antibodies, comprising:
a) the *in vitro* immunisation of human B lymphocytes with an antigen according to the method for *in vitro* immunisation as claimed in claims 1 to 7,
b) the immortalisation of the immunised B lymphocytes obtained in step a), and
c) the recovery of the human antigen-specific antibodies produced by the immortalised B lymphocytes obtained in step b).

9. Method for producing antibodies according to claim 8, comprising an additional step of cloning the immortalised B lymphocytes, between steps b) and c).

10. Method for producing antibodies according to either claim 8 or claim 9, wherein said antigen-specific antibodies are human antibodies of the IgG isotype.

11. Method for producing antibodies according to any of claims 8 to 10, wherein said antigen-specific antibodies are human monoclonal antibodies.
